Europäisches Patentamt

European Patent Office

Office européen des brevets

(11) Veröffentlichungsnummer: **0 309 862 B1**

(12) **EUROPÄISCHE PATENTSCHRIFT**

(45) Veröffentlichungstag der Patentschrift: **12.08.92**

(21) Anmeldenummer: **88115368.8**

(22) Anmeldetag: **20.09.88**

(51) Int. Cl.⁵: **C12N 15/52**, C12N 15/82

(54) **Stilbensynthase-Gen.**

(30) Priorität: **30.09.87 DE 3733017**

(43) Veröffentlichungstag der Anmeldung:
**05.04.89 Patentblatt 89/14**

(45) Bekanntmachung des Hinweises auf die
Patenterteilung:
**12.08.92 Patentblatt 92/33**

(84) Benannte Vertragsstaaten:
**BE CH DE FR GB IT LI NL**

(56) Entgegenhaltungen:

**THE JOURNAL OF BIOLOGICAL CHEMISTRY,
Band 259, Nr. 11, 10. Juni 1984, USA - A.
SCHÖPPNER et al. "Purification and Properties of a Stilbene Synthase from Induced
Cell Suspension Cultures of Peanut" Pages
6806-6811**

(73) Patentinhaber: **BAYER AG**

**W-5090 Leverkusen 1 Bayerwerk(DE)**

(72) Erfinder: **Schröder, Gudrun, Dr.
Sonnhalde 71
W-7803 Gundelfingen(DE)**
Erfinder: **Schröder, Joachim, Prof. Dr.
Sonnhalde 71
W-7803 Gundelfingen(DE)**
Erfinder: **Hain, Rüdiger, Dr.
Talstrasse 53a
W-4018 Langenfeld(DE)**
Erfinder: **Schreier, Peter Helmut, Dr.
Dasselstrasse 16
W-5000 Köln 1(DE)**

**Beschreibung**

Die vorliegende Erfindung betrifft das aus Pflanzen isolierte Gen für Stilbensynthase und seine Verwendung zur Transformation von Vektoren, Wirtsorganismen und Pflanzen sowie zur Erzeugung von Pflanzen, welche eine erhöhte Resistenz gegenüber Schädlingen aufweisen.

Der Begriff Stilbene beschreibt eine Gruppe von chemischen Substanzen, welche in Pflanzen vorkommen und als gemeinsame Grundstruktur das Stilbengerüst (trans-1,2-Diphenylethylen) enthalten. Dieses Grundgerüst kann durch die Addition weiterer Gruppen ergänzt werden. Zwei wichtige Stilbene sind das 3,5-Dihydroxy-stilben (Pinosylvin) und das 3,3′,5-Trihydroxy-stilben (Resveratrol).

Stilbene wurden in bestimmten Bäumen (Angiospermen, Gymnospermen), jedoch auch in einigen krautigen Pflanzen gefunden (in Arten der Familien Myrtaceae, Vitaceae und Leguminosae). Stilbene sind toxisch für Schädlinge, insbesondere für Pilze, Bakterien und Insekten und sind geeignet, diese Schädlinge abzuwehren. In dieser Funktion wird auch ihre größte biologische Bedeutung gesehen. Besonders in krautigen Pflanzen ist es häufig so, daß Stilbene in gesundem Gewebe nur in sehr geringen Konzentrationen vorliegen, daß aber nach Infektion oder Verwundung sehr hohe Menge an Stilbenen an der Infektions-Stelle neu gebildet werden. Diese erhöhte Konzentration korreliert mit erhöhter Resistenz der Pflanzen, die Stilbene synthetisieren können, gegen die Schädlinge. Die Fähigkeit der Synthese dieser Substanzen wird als wichtiger Abwehrmechanismus angesehen. Leider haben nur wenige Nutzpflanzen die Fähigkeit Stilbene zu bilden, bzw. in einem Maße zu erzeugen, welches ihnen eine ausreichende Resistenz gegen Schädlinge verleiht.

Der Schlüssel für die Bildung aller Stilbene ist die Synthese des Grundgerüstes. Bisher wurden im wesentlichen zwei Enzym-Typen beschrieben (Resveratrolsynthase und Pinosylvinsynthase), die beide als Stilbensynthasen bezeichnet werden, da sie beide das Stilben-Grundgerüst synthetisieren. Am besten charakterisiert wurde bisher die Resveratrolsynthase aus Erdnuß (Arachis hypogea); die Eigenschaften dieses Enzymes sind weitgehend bekannt (Schöppner und Kindl, 1984). Sowohl Pinosylvin als auch Resveratrol, die einfachsten Stilbene, wirken generell toxisch, vorzugsweise mikrobizid, insbesondere fungistatisch, auf infizierende Schad-Organismen. Stilbensynthasen benutzen als Substrate Malonyl-CoA und Cinnamoyl-CoA oder Coumaroyl-CoA, also Substanzen, die in jeder Pflanze vorkommen, da sie auch in der Biosynthese anderer wichtiger Pflanzeninhaltsstoffe verwendet werden (z.B. Flavonoide, Blütenfarbstoffe). Zum Thema Stilbene und Stilbensynthase kann auf die folgende Literatur verwiesen werden: Hart, J.H. (1981) Annu. Rev. Phytopathology 19, 437-458; Hart, J.H., Shrimpton, D.M. (1979) Phytophathology 69, 1138-1143; Kindl, H. (1985) in: Biosynthesis and Biodegradation of Wood Components. Ed. Higuchi, T., Academic Press, Inc., pp. 349-377 und Schöppner, A.; Kindl, H. (1984) J. Biol. Chem. 259, 6806-6811.

Ein großer Teil der Welternte von Kulturpflanzen wird ständig durch Schädlinge vernichtet (1967 betrug der Verlust an potentieller Ernte 35 %; vgl. Chemistry of Pesticides, herausgegeben von K.H. Büchel, John Wiley & Sons, New York, 1983, Seite 6). Es besteht somit ein dringendes Bedürfnis alle Möglichkeiten zu erforschen und nutzbar zu machen, welche geeignet sind, den Schädlingsbefall bei Kulturpflanzen zu vermindern oder zu verhindern.

Es wurde nun das neue Gen für Stilbensynthase ("Stilbensynthase-Gen") isoliert, welches in die Erbmasse (das Genom) von Pflanzen eingebaut werden kann, die keine Stilbene oder nur unzureichend Stilbene erzeugen, wodurch eine erhöhte Resistenz dieser Pflanzen gegen Schädlinge hervorgerufen werden kann.

Die erfindungsgemäßen Stilbensynthese-Gene entsprechen dem Stilbensynthase-Gen, welches im Plasmid pGS 828.1 enthalten ist und schließen die davon abgeleiteten DNA-Sequenzen mit Stilbensynthase-Aktivität ein.

Unter Stilbensynthase-Gen soll jede Nukleinsäure (DNA) verstanden werden, die nach ihrer Transkription in RNA und Translation in Protein (in einer geeigneten Umgebung) die Bildung eines Enzyms bewirkt, welches die Eigenschaften einer Stilbensynthase (enzymatische Synthese von Stilben in einer geeigneten Umgebung) besitzt, wobei diese Nukleinsäure aus ihrer natürlichen Umgebung isoliert vorliegt oder in einen Vektor integriert ist oder in einer prokaryontischen oder eukaryontischen DNA als "fremde" DNA oder als "zusätzliche" DNA enthalten ist.

Wenn das Stilbensynthase-Gen an seinem Anfang und/oder Ende noch DNA-Sequenzen enthält, die die Funktion des Gens nicht oder nicht wesentlich behindern, wird im folgenden auch von "Gen-Einheit" gesprochen. Diese Gen-Einheiten entstehen, z.B. durch das Herausschneiden mit Restriktionsenzymen (z.B. partielle Spaltung mit EcoR I und Hind III), da keine Schnittstellen für übliche Restriktionsenzyme exakt am Beginn und am Ende des Gens vorliegen.

Das Stilbensynthase-Gen (bzw. die Gen-Einheit) kann in der Form vorliegen, wie es im Genom von Pflanzen enthalten ist ("genomische" Form, einschließlich nicht Stilbensynthase kodierender und/oder nicht regulatorisch wirkender Sequenzen (wie Introns) oder in einer Form, welche der cDNA ("copy" DNA) entspricht, die über mRNA mit Hilfe von Reverse-Transkriptase/Polymerase erhältlich ist (und keine Introns mehr enthält).

Im erfindungsgemäßen Stilbensynthase-Gen bzw. der Gen-Einheit) können DNA-Abschnitte durch im wesentlichen gleichwirkende andere DNA-Abschnitte ersetzt sein. Auch kann es an den Enden solche DNA-Sequenzen tragen, welche für die Handhabung des Gens (bzw. der Gen-Einheit) jeweils angepaßt sind (z.B. "Linker").

Im vorliegenden Zusammenhang soll unter "fremder" DNA, solche DNA verstanden werden, welche in einem bestimmten prokaryontischen oder eukaryontischen Genom nicht natürlich vorkommt, sondern erst durch Eingriffe durch den Menschen in dieses Genom aufgenommen wird. "Zusätzliche" DNA soll solche DNA sein, welche in dem jeweiligen prokaryontischen oder eukaryontischen Genom zwar natürlich vorkommt, jedoch in zusätzlicher Menge durch Eingriffe durch den Menschen in dieses Genom aufgenommen wird. Die "fremde" DNA oder "zusätzliche" DNA kann je nach Bedarf und Art des vorliegenden Falles in einem oder mehreren Exemplaren eingebaut werden.

Stilbensynthase, welche unter Mitwirkung des erfindungsgemäßen Stilbensynthase-Gens (bzw. der Gen-Einheit) in Pflanzen oder Pflanzenzellen gebildet wird, bedeutet jedes Enzym, welches die Bildung von solchen pflanzlichen Abwehrstoffen gegen Schädlinge (Phytoalexine) bewirkt, die das Stilbengegerüst aufweisen.

Bevorzugte Stilbene sind Pinosylvin (3,5-Dihydroxy-stilben), Pterostilben (3,5-Dimethoxy-4$'$-hydroxystilben) und Resveratrol (3,3$'$,5-Trihydroxy-stilben), wobei Pinosylvin und Resveratrol besonders bevorzugt sind und Resveratrol ganz besonders bevorzugt ist.

Wie bereits erwähnt, werden Stilbene in bestimmten Baumarten sowie in einer Reihe von weiteren, vorzugsweise dikotyledonen (zweikeimblättrigen), Pflanzen gefunden. Als erfindungsgemäßes Stilbenzynthase-Gen wird das Stilbensynthase-Gen bevorzugt, welches aus Gymnospermen, vorzugsweise Pinus, aus Angiospermen, vorzugsweise dikotylen (zweikeimblättrigen) Pflanzen, insbesondere aus Erdnuß (Arachis hypogaea) und Wein (Vitis) und ganz besonders bevorzugt aus Erdnuß isoliert werden kann.

Besonders bevorzugt wird als erfindungsgemäßes Stilbensynthase-Gen das Stilbensynthase-Gen, welches als Gen-Einheit im Plasmid pGS 828.1 (welches weiter unten näher beschrieben wird) vorliegt, sowie die im wesentlichen gleichwirkenden DNA-Sequenzen.

Das Stilbensynthase Gen besteht aus den 5$'$- und 3$'$- untranslatierten Regionen und einer kodierenden Region und liegt auf einem DNA-Fragment von (ca) 6.7 kbp (Gen- Einheit). Die Gen-Einheit weist (3) EcoRI, (3) HindIII und (1) PstI Schnittstellen auf. Sie kann durch partielle Spaltung mit EcoRI und HindIII aus dem Plasmid pGS 828.1 gewonnen werden.

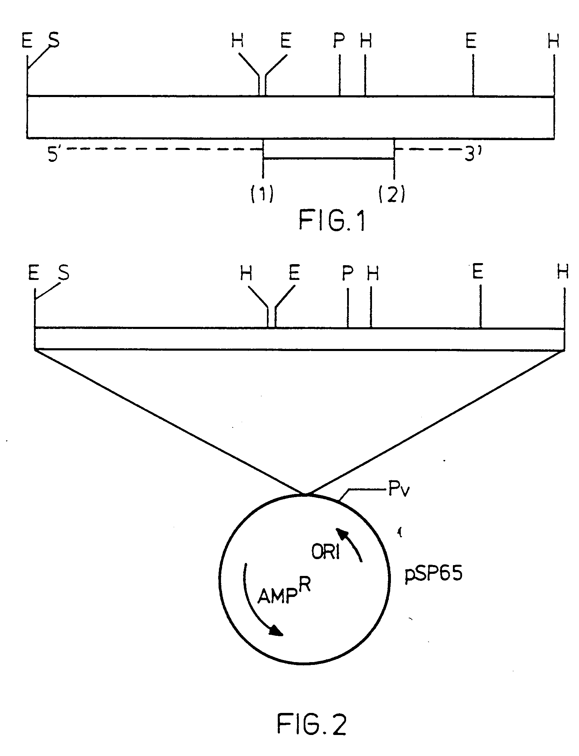

Der 5$'$- regulatorische Teil liegt neben den ersten 7 Kodonen der Protein kodierenden Region auf einem ca. 3.3kbp großen EcoRI Fragment und geht dem Rest der kodierenden Region voraus. Diese Region besteht aus 1540 bp und enthält ein Intron von 369bp und eine HindIII Schnittstelle. Die nachgeschaltete 3$'$-untranslatierte Region ist vollständig vorhanden und wird durch eine EcoRI Schnittstelle (siehe Fig. 1) begrenzt. Die Gen-Einheit, in dem Vektor pSP 65 kloniert (Plasmid pGS 821.1), enthält zwei interne EcoRI eine PstI und zwei HindIII Schnittstellen und ist durch die SstI Schnittstelle (unmittelbar neben der EcoRI Schnittstelle in Richtung Stilbensynthase-Gen) im Polylinker des pSP65 Plasmids am 5$'$-Ende und durch die HindIII Schnittstelle am 3$'$-Ende begrenzt. Die Gen-Einheit kann besonders günstig mit Hilfe von SstI und PvuII aus pGS 828.1 entnommen werden (Fig. 2), da beide Schnittstellen nur je einmal vorkommen. Die PvuII Schnittstelle liegt außerhalb der eigentlichen Gen-Einheit. Dies hat aber auf die Expression des Gens in transgenen Pflanzen keinen Einfluß und der Abschnitt bis zur HindIII-Schnittstelle kann wenn gewünscht mit den üblichen Methoden entfernt werden.

Der Escherichia coli Stamm Nurdug 2010, der das Plasmid pGS 828.1 enthält, wurde bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt und hat die Hinterlegungsnummer DSM 4243 (Hinterlegungsdatum: 17. September 1987).

Dieser Stamm sowie seine Mutanten und Varianten sind ebenfalls Teil der vorliegenden Erfindung. Das in diesem Wirt hinterlegte Plasmid pGS 828.1 kann in üblicher Weise durch die Vermehrung des Stammes leicht in den benötigten Mengen gewonnen werden.

Erfindungsgemäß besonders bevorzugt wird das Stilbensynthase-Gen bzw. die Gen-Einheit, welche die weiter unter aufgeführte (vorgeschlagene) Stilbensynthase codierende DNA-Sequenz ("DNA-Sequenz" (proteinkodierende Region und Intron) des Stilbensynthase-Geneinheit aus pGS 828.1") mit oder ohne das Intron oder im wesentlichen gleichwirkende Sequenzen dieser DNA-Sequenz enthält. Sie ist ebenfalls ein Teil der vorliegenden Erfindung. Darüber hinaus ist auch jede DNA, welche künstlich also nicht im

3

wesentlichen biologisch erzeugt ist, und diese DNA-Sequenz ganz oder teilweise oder die im wesentlichen gleichwirkenden DNA-Sequenzen enthält, Teil der vorliegenden Erfindung.

Im wesentlichen gleichwirkende Sequenzen bedeutet, daß an einer oder mehreren Stellen DNA oder DNA-Sequenzen durch andere DNA oder DNA-Sequenzen ausgetauscht sind, welche jedoch das Ergebnis nicht wesentlich verändern.

Funktionell vollständige Gene, wie das erfindungsgemäße Stilbensynthase-Gen, bestehen aus einem regulatorisch wirkenden Teil (insbesondere Promotor) und dem Strukturgen, welches das Protein Stilbensynthase kodiert.

Beide Genteile können unabhängig voneinader verwendet werden. So ist es möglich, dem regulativ wirkenden Teil eine (vom Stilbensynthase-Gen abweichende) andere DNA-Sequenz nachzuschalten, welche nach dem Einbau in das Pflanzengenom exprimiert werden soll. Da nur wenige isolierte Promotoren bekannt sind, welche ihre Wirkung in Pflanzen bzw. Pflanzenzellen entfalten können, stellt der Promoter des Stilbensynthase-Gens, welcher Bestandteil der vorliegenden Erfindung ist, ein wertvolles Hilfsmittel bei der Erzeugung transformierter Pflanzen bzw. Pflanzenzellen dar. Er kann mit Hilfe der SstI Schnittstelle und der EcoRI Schnittstelle am Beginn der codierenden Region isoliert und mit den üblichen Methoden einem anderen Gen vorgeschaltet werden.

Ebenso ist es möglich, dem Stilbensynthase-Struktur-Gen einen "fremden" regulatorisch wirkenden Teil vorzuschalten. Dies könnte vorteilhaft sein, wenn bei bestimmten Pflanzen nur bestimmte (z.B. pflanzeneigene) regulatorisch wirkende Gene ausreichend wirksam werden können. Das Stilbensynthase-Struktur-Gen (vorzugsweise die weiter unten aufgeführte Stilbensynthase codierende DNA-Sequenz, mit oder ohne die Intron-Sequenzen, und deren im wesentlichen gleichwirkende Sequenzen) stellt somit eine wertvolle, selbstständig einsetzbare Einheit dar und ist, wie bereits dargelegt, ebenfalls Teil der vorliegenden Erfindung. Das erfindungsgemäße Stilbensynthase-Gen kann nach den üblichen Methoden in den regulatorisch wirkenden Teil und das Struktur-Gen getrennt werden. Bevorzugt wird das vollständige erfindungsgemäße Stilbensynthase-Gen bzw. die Gen-Einheit verwendet.

Mit Hilfe der üblichen Methoden ist es möglich, das Stilbensynthase-Gen bzw. die Gen-Einheit oder seine Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. Die so "modifizierte" DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Das Stilbensynthase-Gen bzw. die Gen-Einheit und/oder seine Teile sowie die modifizierte DNA können als "fremde" oder "zusätzliche" DNA in Vektoren (insbesondere Plasmiden, Cosmiden oder Phagen), in transformierten Mikroorganismen (vorzugsweise Bakterien, insbesondere Gram-negativen Bakterien, wie E. coli) sowie in transformierten Pflanzenzellen und Pflanzen bzw. in deren DNA enthalten sein. Solche Vektoren, transformierte Mikroorganismen (die auch diese Vektoren enthalten können) sowie die transformierten Pflanzenzellen und Pflanzen und deren DNA stellen Bestandteile der vorliegenden Erfindung dar.

Als Schädlinge, gegen welche mit Hilfe des erfindungsgemäßen Stilbensynthase-Gens Resistenzen, bzw. erhöhte Resistenzen erzielt werden können, seien tierische Schädlinge, wie Insekten, Milben und Nematoden sowie mikrobielle Schädlinge, wie phytopathogene Pilze und Bakterien genannt. Besonders hervorgehoben werden mikrobielle Schädlinge, insbesondere phytopathogene Pilze.

Zu den schädlichen Insekten gehören insbesondere Insekten der Ordnungen:
Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere:
Tarsonemus spp., Panonychus spp. und Tetranychus spp.

Zu den schädlichen Nematoden gehören insbesondere:
Pratylenchus spp., Heterodera spp. und Meloidogyne spp.

Zu den mikrobiellen Schädlingen gehören insbesondere die phytopathogenen Pilze:
Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

4

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea
(Konidienform: Drechslera, Syn: Helminthosporium);

Cochliobolus-Arten, wie beispielsweise Cochliobolus sativus
(Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocerco sporella herpotrichoides. Weiterhin sei Helminthosporium carbonum aufgeführt.

Zu den Pflanzen, welchen durch den Einbau (Transformation) des erfindungsgemäßen Stilbensynthase-Gens bzw. der Gen-Einheit Resistenz bzw. eine erhöhte Resistenz gegenüber den obigen Schädlingen verliehen werden kann, gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernde Pflanzen, z.B. Getreide (insbesondere Weizen, Roggen, Gerste, Hafer, Hirse, Reis und Mais), Kartoffel, Leguminosa wie Hülsenfrüchte und insbesondere Alfalfa, Sojabohnen, Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen, wie Rauwolfia und Digitalis. Besonders bevorzugt seien Kartoffel, Tomaten, Wein und Leguminosen genannt.

Wie bereits angedeutet, wird erfindungsgemäß das Stilbensynthase-Gen bzw. die Gen-Einheit ein- oder mehrfach (an gleichen oder verschiedenen Stellen des Genoms) in das natürliche pflanzliche Genom eingebaut. Bei Pflanzen, welche bereits über die Fähigkeit der Stilbensynthese verfügen, kann der Einbau eines oder mehrerer erfindungsgemäßer Stilbensynthasegene zu einem erheblich verbesserten Resistenzverhalten führen. Gegebenenfalls wird nur das erfindungsgemäße Strukturgen verwendet, wobei ein evtl. aus der jeweiligen Pflanze isoliertes regulatorisches Gen vorgeschaltet wird.

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze, erhöhte Resistenzen aufweisen.

Die vorliegende Erfindung betrifft somit auch ein Verfahren zur Herstellung transformierter Pflanzenzellen (einschließlich Protoplasten) und Pflanzen (einschließlich Pflanzenteile und Samen), welches dadurch gekennzeichnet, ist daß man

(a) ein oder mehrere Stilbensynthase-Gene bzw. Gen-Einheiten und/oder Teile des Stilbensynthase-Gens bzw. der Gen-Einheit und/oder erfindungsgemäß modifizierte DNA in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls

(c) von den so erhaltenen transformierten Pflanzen die gewünschten Pflanzenteile (einschließlich Samen) gewinnt.

Die Verfahrensschritte (a), (b) und (c) können nach bekannten Verfahren und Methoden in üblicher Weise durchgeführt werden.

Transformierte Pflanzenzellen (einschließlich Protoplasten) und Pflanzen (einschließlich Pflanzenteile und Samen), welche ein oder mehrere Stilbensynthase-Gene bzw. Gen-Einheiten und/oder Teile des Stilbensynthase-Gens bzw. der Gen-Einheiten als "fremde" oder "zusätzliche" DNA enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung des Stilbensynthase-Gens bzw. der Gen-Einheit und/oder seiner Teile und/oder der erfindungsgemäß modifizierten DNA und/oder der erfindungsgemäßen Vektoren und/oder der erfindungsgemäßen transformierten Mikroorganismen zur Transformation von Pflanzenzellen (einschließlich Protoplasten) und Pflanzen (einschließlich Pflanzenteilen und Samen) sowie die

(b) Verwendung der erfindungsgemäßen transformierten Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial und allgemein die

(c) Verwendung des erfindungsgemäßen Stilbensynthase-Gens bzw. der Gen-Einheit und/oder seiner Teile und/oder der erfindungsgemäßen modifizierten DNA zur Bekämpfung von Schädlingen.

Eine Anzahl verschiedener Methoden steht zur Verfügung, das Stilbensynthase-Gen bzw. die Gen-Einheit oder seine Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Das genetische Material, welches für Stilbensynthase kodiert, wird in die T-DNA von geeigneten Ti-Pasmiden eingesetzt (z.B. Zambryski et al. 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält und Pflanzenprotoplasten (z.B. Davey et al. 1980; Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Formm et al. 1986).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) wird die aus (ca) 6,7 kb bestehende DNA-Einheit aus pGS 828.1, charakterisiert durch die entsprechenden internen EcoRI, HindIII bzw. PstI Schnittstellen (siehe Figur 1) in einen geeigneten intermediaeren E.coli Vektor z.B. pGV700, pGV710, (vergl. EP-A-116 718; Deblaere et al. 1986) bzw. vorzugsweise Derivaten davon, die zusätzlich ein Reportergen wie z.B. nptII (Herrera-Estrella et al. 1983) oder hpt (Van den Elzen et al 1986) enthalten, kloniert. Hierzu kann auch der mit Hilfe von SstI und PvuII aus pGS 828.1 entnommene DNA-Abschnitt verwendet werden.

Der Escherichia coli Stamm AZ 4, der den Vektor pGV 710 in leicht isolierbarer Form enthält, wurde bei der Deutschen Sammlung von Mikroorganismen (DSM), Grisebachstraße 8, D-3400 Göttingen, Bundesrepublik Deutschland in Übereinstimmung mit den Bestimmungen des Budapester Vertrages über die internationele Anerkennung der Hinterlegung von Mikroorganismen für die Zwecke von Patentverfahren hinterlegt und hat die Hinterlegungsnummer DSM 3164.

Das so konstruierte Plasmid wird auf Agrobacterium tumefaciens, das z.B. pGV 3850 bzw. Derivate davon enthält (Zambryski et al. 1983) mit üblichen Methoden (z.B. Van Haute et al. 1983) übertragen. Alternativ dazu kann die Stilbensynthase-Geneinheit in einem binaeren Vektor (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Stilbensynthase-Geneinheit in einer auf Pflanzen transferierbaren Form enthält wird im weiteren zur Pflanzentransformation verwendet.

In einer weiteren bevorzugten Ausführungsform wird das isolierte Plasmid pGS 828.1 gegebenenfalls zusammen mit einem anderen Plasmid, das ein Reportergen für Pflanzenzellen, z.B. für Kanamycin-Resistenz (z.B. Herrera-Estrella et al. 1983) oder eine Hydromycin-Resistenz (van den Elzen, 1986) enthält, vorzugsweise pLGV neo 2103 (Hain et al. 1985), pLGV 23 neo (Herrera-Estrella 1983), pMON 129 (Fraley R.T. et al., Proc. National Acad. Sci. USA 80, 4803 (1983), pAK 1003, pAK 2004 (Velten J. et al., EMBO Journ. Vol. 3, 2723 (1984) oder pGSST neo 3 (pGSST3) (EP-A-189 707), in üblicher Weise durch direkten Gentransfer auf Pflanzenprotoplasten übertragen (z.B. Hain et al 1985). Dabei können das bzw. die Plasmide in zirkulärer, vorzugsweise jedoch in linearer Form, vorliegen. Bei der Verwendung eines Plasmids mit Reportergen werden Kanamycin-resistente Protoplasten dann auf Expression von Stilbensynthase überprüft. Im anderen Fall (ohne Reportergen) werden die resultierenden Kalli auf die Expression des Stilbensynthase-Gens geprüft (Screening mit üblichen Methoden).

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro ((Reiss et al. 1984; Schreier et al. 1985) oder durch die Expression der Nopalinsynthase (nach Aerts et al. 1983) oder Stilbensynthase durch Northern-Blot-Analyse und Western Blot-Analyse nachgewiesen. Die Stilbensynthase und die Stilbene können auch in bekannter Weise mit Hilfe spezifischer Antikörper in transformierten Pflanzen nachgewiesen werden. Stilbensynthase kann auch durch Enzymaktivitätstest nachgewiesen werden (Rolfs et al., Plant Cell Reports 1, 83-85, 1981).

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche das erfindungsgemäße Stilbensynthase-Gen bzw. die Gen-Einheit enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung bei transformierten Pflanzen und Pflanzenzellen verwendet werden können und ist somit ebenfalls Teil der vorliegenden Erfindung.

Im vorliegenden Zusammenhang bedeutet der Ausdruck "im wesentlichen gleichwirkende DNA-Sequenzen", daß die Erfindung auch solche Modifikationen umfaßt, bei welchen die Funktion des Stilbensynthase-Gens und seiner Teile nicht derart beeinträchtigt ist, daß Stilbensynthase nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nukleinsäuren erfolgen.

Bei den erfindungsgemäß verwendbaren Mikroorganismen bedeutet "Mutanten" und "Varianten" solche modifizierten Mikroorganismen, welche noch die für die Ausführung der Erfindung wesentlichen Merkmale aufweisen, insbesondere die jeweiligen Plasmide enthalten.

Die vorliegende Erfindung soll anhand der folgenden beispielhaften Ausführungen näher erläutert werden:

## 1. Isolierung des Gens für Stilbensynthase

Zellkulturen aus Ernußpflanzen (Arachis hypogaea) enthalten das Gen für Stilbensynthase, welche insbesondere die Bildung von Resveratrol Synthase (Größe des Proteins 43 000 D; Reaktion mit spezifischem Antiserum) bewirkt.

Die Erdnuß-Zellkulturen, die Regulation und Eigenschaften der Stilbensynthase, das Antiserum gegen das Protein, und die Messung der Enzymaktivität sind beschrieben (Rolfs, C.H., Fritzemeier, K.H., Kindl, H., Plant Cell Reports 1, 83-85, 1981; Fritzemeier, K.H. Rolfs, C.H., Pfau, J., Kindl, H., Planta 159, 25-29, 1983; Schöppner, A., Kindl, H., J. Biol. Chem. 259, 6806-6811, 1984; siehe auch die Zusammenfassung: Kindl, H., in: biosynthesis and Biodegradation of Wood Components, Ed. Higuchi, T., Academic Press, Inc., pp. 349-377, 1985).

Das folgende Schema faßt die Expression des Gens und die Isolierung des Gens für Stilbensynthase zusammen. Bei der Durchführung wurden die bekannten Verfahren und Methoden der Molekularbiologie verwendet, wie sie beispielsweise in folgendem Handbuch detailliert beschrieben werden: Maniatis, T., Fritsch, E.F., Sambrook, J.: Molecular Cloning: A Laboratory Manual; Cold Spring Harbor Laboratory, 1982.

Schema zur Expression des Stilbensynthase Gens sowie zu seiner Isolierung aus Pflanzenzellen

Das Gen für Stilbensynthase und seine Expression in Pflanzenzellen

Genom im Zellkern (enthält Gen für Stilbensynthase

→ Transkription

mRNA-Population (enthält mRNA für Stilbensynthase, Größe 1600 Nucleotide)

→ Translation

Protein-Population enthält Stilbensynthase-Protein, definiert durch:
- Enzymaktivität
- Proteingröße (43000 D)
- Reaktion mit spezifischem Antiserum

B. Gen-Bibliothek (in E. coli)

A. cDNA-Bibliothek (in E. coli)

Isolierung des Gens ===================

Gen für Stilbensynthase Identifizierung:
- Hybridisierung mit Stilbensynthase mRNA
- Hybridisierung und Vergleich mit Stilbensynthase cDNA

Stilbensynthase cDNA Identifizierung:
- Hybridisierung an Stilbensynthase mRNA
- Isolierung reiner Stilbensynthase mRNA durch Hybridisierung

→ Translation (in vitro)

Stilbensynthase-Protein, definiert durch:
- Proteingröße (43000 D)
- Reaktion mit spezifischem Antiserum, Enzymaktivität

A. Isolierung und Identifizierung spezifischer cDNA-Klone für Stilbensynthase.

Es wird zunächst Poly(A)-haltige RNA aus Erdnußzellkulturen isoliert, welche Stilbensynthase synthetisieren. Die mRNA wird dann mit Hilfe von Reverse-Transkriptase und E. coli DNA-Polymerase in DNA umgeschrieben; die DNA wird in Plasmid-Vektor pINIIA mit Hilfe von Linkern kloniert und in E. coli-Stamm JA221 vermehrt (Nakamura, K., Inouye, M., EMBO J. 1, 771-775, 1982). Dieser Prozeß ergibt eine "cDNA-Bibliothek": Sie repräsentiert, kloniert als DNA in E. coli-Plasmiden, die mRNA-Population der Erdnußzellen. Sie enthält die Nucleinsäure, die für Stilbensynthase kodiert, und diese wird durch folgende Schritte identifiziert:

(i) die spezifische cDNA hybridisiert mit Stilbensynthase-mRNA,

(ii) durch Hybridisierung mit der cDNA wird eine mRNA isoliert, die bei in vitro-Übersetzung ein Protein ergibt, welches identisch ist mit Stilbensynthase (Proteingröße 43000 D; Reaktion des Proteins mit Antiserum, welches spezifisch mit Stilbensynthase-Protein reagiert).

Diese Kriterien definieren auf der cDNA-Ebene eindeutig die Nucleinsäure, welche für das Stilbensynthase-Protein kodiert.

B. Isolierung des Gens für Stilbensynthase.

Es wird zunächst eine "Gen-Bibliothek" für Ernußzellen angelegt: Genomische DNA aus angereicherten Zellkernen (Bedbrook, J., Plant Molecular Biology Newsletter 2, 24, 1981) wird mit dem Restriktions-Enzym SauIIIa so geschnitten, daß DNA-Fragmente mit einer Durchschnittslänge von 10 000-25 000 Nukleotidpaaren entstehen. Diese Fragmente werden in die BamHI-Stelle von Lambda-Phage EMBL3 kloniert (Frischauf et al., J. Mol. Biol. 170, 827-842, 1983), und die Phagen werden in E. coli vermehrt. Die Gesamtheit der Phagen-Population enthält, kloniert in Teilstücken, die gesamte genomische DNA der Erdnußzellen, und damit auch das Gen für Stilbensynthase.

Das Gen für Stilbensynthase, seine mRNA und die Stilbensynthase-cDNA enthalten gleiche Nucleinsäuresequenzen, da sie voneinander abgeleitet sind (Gen→mRNA→cDNA). Dies bedeutet, daß das Gen für Stilbensynthase durch spezifische Hybridisierung mit Stilbensynthase-cDNA oder -mRNA identifizierbar ist. Die Phagen mit dem Gen werden durch Hydridisierung identifiziert, dann isoliert und vermehrt. Die in diesem Phagen klonierte genomische DNA aus Erdnußzellen wird weiter durch Analyse mit verschiedenen Restriktionsenzymen kartiert, und die Position des Stilbensynthase-Gens wird durch weitere Hybridisierungs-Experimente mit der cDNA festgelegt. Schließlich wird die Gen-Einheit durch partiellen Verdau mit EcoRI und HindIII aus dem Phagen herausgeschnitten, im entsprechend geschnittenen Plasmid-Vektor pSP65 kloniert (Fa. Amersham Buchler GmbH & Co. KG, Braunschweig, Bundesrepublik Deutschland), und als rekombinantes Plasmid vermehrt. Dieses Plasmid wird als pGS 828.1 bezeichnet.

2. Beschreibung des Plasmids pGS 828.1 (vgl. Fig. 1 - Fig. 2)

Das Plasmid besteht aus zwei Komponenten:

(i) Gen-Einheit Stilbensynthase: Das gesamte Gen, bestehend aus Struktur-Gen und Regulator-Anteil (beide aus Erdnußzellen) befindet sich auf einer Nucleinsäure, die als DNA-Fragment von (ca) 6700 Nukleotidpaaren durch partielle Spaltung mit den Restriktionsenzymen EcoRI und HindIII aus dem Plasmid herausgeschnitten wird. Die Gen-Einheit enthält Schnittstellen für die Restriktionsenzyme EcoRI, HindIII, und PstI.

(ii) Vektor-Plasmid: Die Gen-Einheit ist in Vektor pSP65 kloniert. Die Größe des Vektors ist 3000 Nukleotidpaare. Er trägt das Gen für Ampicillin-Resistenz, d.h. E. coli-Zellen mit diesem Plasmid wachsen in Nährmedien, die das Antibiotikum Ampicillin enthalten. Ori: Bezeichnung für Sequenzen, die für die Vermehrung des Plasmids in E. coli notwendig sind.

Das Plasmid trägt ein Gen für Ampicillin-Resistenz und enthält 9350 Nucleotidpaare (9,35 kBp). Es kann in E. coli Zellen, welche pGS 828.1 enthalten (E. coli Nurdug 2010, in üblicher Weise vermehrt werden.

Bevorzugtes Nährmedium für E. coli-Zellen (z.B. JA221, Nakamura, K., Inouye, M., EMBO J. 1, 771-775, 1982) welche pGS 828.1 enthalten (E. coli Nurdug 2010):

| Bacto-Pepton* | 10 g |
|---|---|
| Hefeextrakt | 5 g |
| NaCl | 5 g |
| Agar | 20 g |
| $H_2O$ | 1 l |
| pH 7,5 | |
| Fermentation: | 37° C, aerob |

(* Bacto ist ein Warenzeichen der Fa. DIFCO Lab. Detroit, USA).

## 3. Transformation von Tabak

a) Kultur von Tabaksprossen und Isolierung von Tabakprotoplasten:

Nicotiana tabacum (Petit Havanna SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26° C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x $10^5$/ml verdünnt. Die Protoplastenkonzentration wird in einer Zählkammer bestimmt.

b) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von 1-2 x $10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin) 2 Tage im Dunkeln und ein bis zwei Tage lang unter Schwachlicht (500 lux) bei 26° C inkubiert. Sobald die ersten Teilungen der Protoplasten auftreten, werden 30 $\mu$l einer Agrobakteriumsuspension in minimal A (Am) Medium (Dichte ca. $10^9$ Agrobakterien/ml) zu 3 ml regenerierenden Protoplasten gegeben. Die Kokulturdauer beträgt 3-4 Tage bei 20° C im Dunkeln. Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x $10^4$/ml in K3 Medium (0,3 m Saccharose) mit 1 mg/l NAA (Naphthyl-1-essigsäure), 0,2 ml/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose Bead Typ Kultur (Shillito et al. 1983) gestartet. Kanamycinresistente Kolonien können 3-4 Wochen nach Beginn der Selektion vom Hintergrund zurückgebliebender Kolonien unterschieden werden.

c) Direkte Transformation von Tabakprotoplasten mit DNA. Calciumnitrat-PEG Transformation.

In einer Petrischale werden ca. $10^6$ Protoplasten in 180 $\mu$l K3 Medium mit 20 $\mu$l wäßriger DNA Lösung welche 0,5 $\mu$g/$\mu$l pGS 828.1 und 0,5 $\mu$g/$\mu$l pLGV neo 2103 (Hain et al. 1985) enthält, vorsichtig gemischt. Anschließend werden 200 $\mu$l Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleiner Menge K3 Medium aufgenommen und wie im nächsten Abschnitt beschrieben kultiviert. Alternativ können die Protoplasten nach Hain et al. 1985 tranformiert werden.

Die Transformation kann auch ohne den Zusatz der 0,5 $\mu$g/$\mu$l pLGV neo 2103 durchgeführt werden. Da in diesem Fall kein Reportergen eingesetzt wird, werden die resultierenden Kalli auf das Vorhandensein der Stilbensynthase-Gen-Einheit mit Hilfe einer Dot-Blot-Hybridisierung überprüft. Als Hybridisierungs-Probe ist ein internes EcoRI-HindIII-Fragment aus pGS 828.1 verwendbar. Selbstverständlich können auch andere Nachweismethoden, wie Test mit Antikörpern oder Feststellung einer Fungizid-Resistenz eingesetzt werden.

d) Kultur der mit DNA inkubierten Protoplasten und Selektion Kanamycin resistenter Kalli:

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. c) werden 1 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

```
     Schema der Selektion kanamycinresistenter Tabak-
     kolonien nach DNA Transformation:


   0,4 M  0,3 M  0,25 M  0,20 M  0,15M  0,10 M  Saccha-
                                                rose im
                                                Flüssig-
                                                medium

   A   E S                                K
  ─────────────────────────────────────────────────────
       1      2       3       4      5       6  Wochen
                                                nach DNA
                                                Aufnahme
     (K3 Medium 1 mg NAA, 0,2 mg Kinetin)


   A = DNA Aufnahme
   E = Einbettung in Agarose
   S = Selektion mit Kanamycin (100 mg/l Kanamycinsulfat)
   K = Kanamycinresistente Kolonien können vom Hintergrund
       eindeutig unterschieden werden
```

e) Regeneration kanamycinresistenter Pflanzen:

Sobald die kanamycinresistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24° C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamycinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

f) Transformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien (ca. $10^9$/ml) (vgl. b) in Am-Medium, siehen unten) für ca. 5 Minuten inkubiert. Die infizierten Blattstücke werden auf MS-Medium (siehe unten) ohne Hormone für 3-4 Tage bei ca. 24° C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 $\mu$g/ml Cefotaxim und 100 $\mu$g/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Stilbensynthase Aktivität auf Transformation getestet weren. Auf diese Weise werden 1-10 % transformierte Sproße erhalten.

Biochemische Nachweismethode der Transformation

Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 $\mu$l des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15 % Essigsäure, 80 % $H_2O$, pH 1,8) getränkt und bei 400 V für 45 Minuten elektrophoretisiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen. Das getrocknete Papier wird unter langwelligem UV-Licht betrachtet und fotografiert. Arginin und Argininderivate werden mit dem Reagenz gelb fluoreszierend gefärbt.

Neomycin-Phosphotransferase (NPT II) Enzymtest:

NPT II Aktivität in Pflanzengewebe wird durch in situ Phosphorylierung von Kanamycin, wie bei Reiss et al. (1984) beschrieben und von Schreier et al. (1985) modifiziert, wie folgt, nachgewiesen. 50 mg Pflanzengewebe werden in 50 $\mu$l Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 $\mu$l des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM $MgCl_2$, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 $\mu$g/ml) und 20-200 $\mu$Ci $^{32}$P ATP (Amersham) enthält, überschichtet. Das Sandwichgel wird 30 Min. bei Zimmertempreratur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak).

4. Transformation von Medicago Sativa (Luzerne)

a) Pflanzenmaterial

Die Pflanze Medicago sativa (Regen S, Klon RA$_3$ Walker et al, 1978) wurde als sterile Sproßkultur auf LS Medium (Linsmaier und Skoog, 1965), im Langtag (16 h Licht, 8 h Dunkelheit) bei 26 ± 2°C kultiviert.

b) Kulturbedingungen

Als Kulturgefäße für Sproßkulturen dienten Glasgefäße (250 ml - 1,5 l) mit lose aufliegenden Glasdek-keln. Für alle anderen Pflanzenkulturen (Embryo, Kallus, Protoplasten) wurden Petrischalen aus Plastik benutzt.

Die Pflanzen und Gewebekulturen bis auf die Protoplasten wurden in Kulturräumen im Langtag (16 h Licht, 8 h Dunkelheit) bei 26 ± 2°C kultiviert. Die Leuchtstoffröhren hatten die Lichtfarbe Universal Weiß (Osram L58W/25). Der Abstand der Röhren von den Kulturen betrug 10-30 cm, das entspricht 1500-4500 Lux Lichtintensität. Die Luftfeuchtigkeit blieb unreguliert. Die Protoplasten wurden in Kulturschränken bei maximal 500 Lux und 26°C kultiviert.

c) Kalluskultur

Kallus wurde von Petiolen der Gewächshauspflanzen induziert. Ca. 5 cm lange Petiolenstücke wurden von den Gewächshauspflanzen mit einem Skalpell abgeschnitten. Die Petiolenstücke wurden zuerst oberflächensterilisiert:
- 1 min in 70% Ethanol
- 10 min in 10% handelsüblichem Desinfektionsmittel (z.B. Dan Klorix)
- 3 Waschungen in sterilem Leitungswasser.

Nach der Sterilisation wurden die Petiolenstücke in 1-1,5 cm lange Stücke geschnitten und in Petrischalen auf festes Agarmedium ausgelegt. Drei verschiedene Medien wurden zur Kallusinduktion und Weiterkultur benutzt:

1. B$_5$h (Atanassov und Brown, 1984)
2. SHR: SH (Shenk und Hildebrandt, 1972) mit 25 $\mu$M (4,655 mg/l) NAA und 10 $\mu$M (2,15 mg/l) Kinetin (Walker und Sato, 1981)
3. B$_5$H$_3$: B$_5$ (Gamborg et al., 1968) mit 2,6 $\mu$M (0,5 mg/l) NAA, 2,2 $\mu$M (0,5 mg/l) BAP, 2,2 $\mu$M (0,5 mg/l) 2,4D (Oelck, Dissertation 1984).

Nach drei Wochen wurden jeweils die äußeren Teile des Kallus mit einem Skalpell abgeschnitten und auf frischem Medium subkultiviert.

d) Kallusregeneration

Die Regeneration von Pflanzen aus Kallus wurde nach dem Protokoll von Stuart und Strickland, 1984 a, b, mit Modifikationen durchgeführt.

Die somatische Embryogenese wurde durch eine Inkubation von Kallusgewebe in flüssigem SH-Medium (Shenk und Hildebrandt, 1972), das 50 $\mu$M (11 mg/l) 2,4D und 5 $\mu$M (1,07 mg/l) Kinetin enthielt, induziert. In einem Erlenmeyerkolben (100 ml in einem 500 ml Kolben) wurden pro ml Medium 30 mg Kallus (Frischgewicht) zugegeben. Die Induktion erfolgte für 3-4 Tage auf einem Schüttler (100 Upm) bei 26°C im Pflanzenkulturraum. Anschließend wurde das Kallusgewege vom Medium auf einem Sieb (850 $\mu$m ) getrennt.

Mit einem Spatel wurde es durch das Sieb gepreßt und kleine Zellhaufen wurden auf einem sich darunter befindenden Sieb mit der Maschenweite 250 $\mu$m$^2$ gesammelt. Pro 100 ml Induktionsmedium wurden die Zellhaufen mit 500 ml SHJ-Medium ohne Hormone (SH) gewaschen. Die Waschlösung wurde durch Abtropfen so weit wie möglich entfernt (ca. 5 min). Das Frischgewicht wurde bestimmt und die Zellhaufen wurden in SH-Medium resuspendiert. 75 mg in 0,5 ml wurden in einer Pipette auf ca. 10 ml festes Regenerationsmedium SHR aufgebracht. Das Regenerationsmedium SHR bestand aus SH-Medium mit 25 mM NH$_4^+$ und 100 mM L-Prolin in 3% Saccharose.

Nach ca. vier Wochen wurden gut entwickelte Embryos mit einer deutlichen Polarität (Keimblattstadium, Dos Santos et al., 1983) auf festes 1/2SH-Medium mit 25 $\mu$M (8,6 mg/l) Gibberilinsäure (GA$_3$) und 0.25 $\mu$M

(0,046 mg) NAA gesetzt. Nach der Bewurzelung und der Entwicklung eines Sprosses mit Blättern wurden die kleinen Pflänzchen auf LS-Medium umgesetzt.

Die Tabelle gibt die Zusammensetzung der Medien $B_5$h, SHJ, SHR, 1/2 SH, LS an. Das flüssige Medium SH entspricht dem SHJ-Medium ohne die Hormone 2,4D und Kinetin. Die Angabe der Mengen ist in mg/l, wenn nichts anderes vermerkt ist.

| Makroelemente | $B_5$h | SHJ | SHR | 1/2SH | LS |
|---|---|---|---|---|---|
| $NH_4NO_3$ | - | - | - | | 1650 |
| $KNO_3$ | 3000 | 2500 | 2500 | 1250 | 1900 |
| $CaCl_2$ $2H_2O$ | 895 | 200 | 200 | 100 | 1900 |
| $MgSO_4$ $7H_2O$ | 500 | 400 | 400 | 200 | 370 |
| $(NH_4)_2SO_4$ | 134 | - | 1651 | - | - |
| $NaH_2PO_4H_2O$ | 156 | - | 407 | - | - |
| $KH_2PO_4H_2O$ | - | - | - | - | 170 |
| $NH_4H_2PO_4$ | - | 300 | - | 150 | - |

| Mikroelemente | | | | | |
|---|---|---|---|---|---|
| $ZnSO_4H_2O$ | 10 | 10 | 10 | 5 | 22,3 |
| $H_3BO_3$ | 3 | 5 | 5 | 2,5 | 6,2 |
| $ZnSO_4$ $7H_2O$ | 1 | 1 | 1 | 0,5 | 8,6 |
| $Na_2MoO_4 2H_2O$ | 0,25 | 0,1 | 0,1 | 0,05 | 0,25 |
| $CuSO_4$ $5H_2O$ | 0,025 | 0,02 | 0,02 | 0,1 | 0,025 |
| $CaCl_2$ $6H_2O$ | 0,025 | 0,1 | 0,1 | 0,05 | 0,025 |
| KJ | 0,75 | 1 | 1 | 0,5 | 0,83 |
| $FeSO_4$ $7H_2O$ | 28 | 15 | 15 | 7,5 | 28 |
| $Na_2EDTA$ | 37 | 20 | 20 | 10 | 37 |

| Vitamine | | | | | |
|---|---|---|---|---|---|
| Thiamine HCL | 10 | 5 | 5 | 2,5 | 0,4 |
| Pyridoxine HCl | 1 | 0,5 | 0,5 | 0,25 | - |
| Nikotinsäure | 1 | 5 | 5 | 2,5 | - |

| | B$_5$h | SHJ | SHR | 1/2SH | LS |
|---|---|---|---|---|---|
| **Aminosäuren** | | | | | |
| L-Glutamin | 800 | - | - | - | - |
| L-Serin | 100 | - | - | - | - |
| L-Prolin | - | - | 5755 | - | - |
| **Andere Bestandteile** | | | | | |
| **u.a. Phytohormone** | | | | | |
| Myo-Inositol | 100 | 1000 | 1000 | 500 | 100 |
| L-Gluthathion | 10 | - | - | - | - |
| Adenine-sulfat | 1 | - | - | - | - |
| 2, 4D | 1 | 11,5 | - | - | - |
| Kinetin | 0,2 | 1,075 | - | - | - |
| GA$_3$ | - | - | - | 8,6 | - |
| NAA | - | - | - | 0,046 | - |
| Sucrose | 30 g | 30 g | 30 g | 15 g | 10 g |
| pH | 5,8 | 5,9 | 5,9 | 5,9 | 5,8 |

**Die Einstellung des pH-Wertes erfolgte mit 1N KOH.**

Die Medien wurden durch Erhitzen im Autoklaven für 17 min bei 121°C sterilisiert. Kinetin, L-Gluthathion und Aminosäuren wurden mit einem Filter sterilisiert und nach dem Erhitzen im Autoklaven dem 60°C warmem Medium zugegeben.

e) Protoplastenkultur

Als Ausgangsmaterial zur Isolierung von Protoplasten dienten die Blätter 2-3 Monate alter steriler Sproßkulturen. Die Ernte erfolgte 2-3 h nach dem Einschalten des Lichtes.
- Zuerst wurden die Blätter in einer Petrischale mit EMI (Atanassov und Brown, 1984) befeuchtet und mit einer neuen Rasierklinge fein zerschnitten.
- 1-1,5 g Blätter wurden dann mit 10 ml Enzymlösung in einer Petrischale (Ø 10 cm) für 3-4 h inkubiert. Die Inkubation erfolgte bei 26°C und schwacher Beleuchtung. Die Freisetzung von Protoplasten aus den Blättern wurde unter dem Mikroskop verfolgt. Alle 30 min wurde die Schale 2-3 mal geschwenkt.

Die Enzymlösung bestand aus einer Mischung von 1:1 aus dem Protoplastenkulturmedium (AP) von Atanassov und Brown (1984) mit den Hormonen 0,2 mg/l 2,4D, 0,5 mg/l Zeatin und 1 mg/l NAA und einer Enzymlösung. Die Enzymlösung (Kao und Michayluk, 1979; modifiziert) bestand aus:
- 200 mg Cellulose Onozuka R10
- 80 mg Macerozyme R10          Serva
- 10 mg Pectolyase Y-23          Sigma
- 540 mg Sorbitol

f) Transformation eines induzierten Kallus

Kallus wurde nach der Methode, die unter Kallusregneration beschrieben ist, zur Embryogenese induziert.

Im Anschluß an die 3-4tägige Inkubation in flüssigem SHJ wurde das Kallusmaterial auf einem Sieb (Maschenweite 100 $\mu$m) mit flüssigem SHR, das keinen Agar und keinen L-Prolin enthielt, gewaschen. Danach wurde das Kallusmaterial in flüssigem SHR aufgenommen. Ca. 1 g Kallusmaterial wurde 10 ml Medium zugesetzt.

Nach der Zugabe der Agrobakterien, welche Stilbensynthase-Gen tragende Ti-Plasmide enthalten, ($2\times10^7$/ml Endkonzentration) wurde das Kallusmaterial auf einem Schüttler (90Upm) für 2-3 Tage bei 26°C inkubiert.

Danach wurde das Material auf einem Sieb (Maschenweite 100 $\mu$m$^2$) mit flüssigem SHR gewaschen.

Die Plattierung (75 mg Kallus/10 ml Medium) erfolgte auf dem normalen festen SHR mit 100 mM L-Prolin. Das Medium in den Platten enthielt neben den selektiven Antibiotika 500 $\mu$g/ml Claforan. Nach vier Wochen wurden die resistenten Strukturen auf frisches antibiotikahaltiges Medium gesetzt, und weitere drei Wochen später wurden sie geteilt und eine Hälfte wurde auf frisches Medium ohne selektive Antibiotika, die andere Hälfte auf antibiotikahaltiges Medium gesetzt.

a) Transformation von Embryos

Die Tansformation von Embryos wurde analog zu der Transformation des induzierten Kallus durchgeführt. Als Ausgangsmaterial wurden 4-5 Wochen alte Embryos verwendet. Sie wurden in einer Petrischale mit einer Rasierklinge fein zerschnitten und dann auf einem Sieb (Maschenweite 100 $\mu$m) mit flüssigem SHR gewaschen. Ca. 1 g zerschnittene Embryos wurden in 10 ml flüssigem SHR aufgenommen. Nach der Zugabe von Agrobakterien (2 $\times$ $10^7$/ml Endkonzentration) erfolgte die Inkubation auf einem Schüttler (90 Upm) für 2-3 Tage bei 26°C. Danach wurden die Embryostücke auf einem Sieb (100 $\mu$m$^2$) mit flüssigem SHR gewaschen. Die Plattierung erfolgte mit einem Spatel auf Platten, die das normale feste SHR mit 100 mM L-Prolin enthielten. Ca. 50-100 mg Embryostücke wurden pro Platte, die 10 ml Medium enthielt, verteilt. Das Medium in den Platten enthielt neben den selektiven Antibiotika 500 $\mu$g/ml Claforan. Drei Wochen nach der Plattierung wurden die sekundären Embryos auf frischen Platten subkultiviert. Gut entwickelte Embryos wurden auf antibiotikafreies 1/2 SH-Medium (Stuart und Strickland, 1984, b) gesetzt, um eine Weiterentwicklung zu Pflanzen zu ermöglichen. Kleine Pflänzchen mit Wurzeln wurden dann auf LS Medium umgesetzt.

5. Transformation von Solanum tuberosum (Kartoffel)

Die Transformation wurde genau nach dem in der EP-A-0 242 246, Seiten 14 bis 15 angegebenen Weise transformiert, wobei die Agrobakterien Ti-Plasmide enthalten, die das Stilbensynthese-Gen tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen (Tabak) wurde die Anwesenheit des Stilbensynthase-Gens durch Southern Blot Analyse bestätigt. Die Expression des Stilbensynthase-Gens wurde durch Northern Blot Analyse, Stilbensynthase und Stilbene mit Hilfe von spezifischen Antikörpern nachgewiesen. Transformierte und nicht-transformierte Pflanzen (zum Vergleich) wurden mit einer Sporensuspension von Botrytis cinera besprüht und nach 1 Woche der Pilzbefall bonitiert. Die transformierten Pflanzen zeigten (gegenüber den nicht transformierten Vergleichspflanzen) eine erhöhte Resistenz gegen Pilzbefall. Entsprechende positive Befunde ergaben sich auch bei Medicago sativa und Kartoffel.

Im Folgenden wird die DNA-Sequenz (Protein kodierende Region und Intron) des Stilbensynthase Gens mit einem Teil der 5$'$- und 3$'$- untranslatierten Regionen aufgeführt, wie sie im Plasmid pGS 828.1 vorliegt. Die Restriktionsschnittstellen für EcoRI, PstI und HindIII werden angegeben. Die entsprechende Proteinsequenz ist im Einbuchstabencode angegeben.

```
                                        E
                                        c
                                        o
                                        R
                                        I

     cacagctaagaaaacATGGTGTCTGTGAGTGGAATTCGCAAGGTTCAAAGGGCAGAAGGT
   1 ---------+---------+---------+---------+---------+---------+ 60
     gtgtcgattcttttcTACCACAGACACTCACCTTAAGCGTTCCAAGTTTCCCGTCTTCCA

                 M  V  S  V  S  G  I  R  K  V  Q  R  A  E  G   -

     CCAGCAACGGTATTGGCGATCGGAACAGCAAATCCACCAAACTGTGTTGATCAGAGTACA
  61 ---------+---------+---------+---------+---------+---------+ 120
     GGTCGTTGCCATAACCGCTAGCCTTGTCGTTTAGGTGGTTTGACACAACTAGTCTCATGT

      P  A  T  V  L  A  I  G  T  A  N  P  P  N  C  V  D  Q  S  T   -

     TATGCAGATTATTATTTTAGAGTAACCAACGGCGAACACATGACTGATCTCAAGAAGAAA
 121 ---------+---------+---------+---------+---------+---------+ 180
     ATACGTCTAATAATAAAATCTCATTGGTTGCCGCTTGTGTACTGACTAGAGTTCTTCTTT

      Y  A  D  Y  Y  F  R  V  T  N  G  E  H  M  T  D  L  K  K   -

     TTTCAGCGCATCTgtatgtattttattaagcgttctatatttgtttatatttaatattt
 181 ---------+---------+---------+---------+---------+---------+ 240
     AAAGTCGCGTAGAcatacataaaaataattcgcaagatataaacaaatataaattataaa

      F  Q  R  I  C

     atcaaaataaaattcgttcttttattttttatattaattaatacaggaataatttaacata
 241 ---------+---------+---------+---------+---------+---------+ 300
     tagttttattttaagcaagaaaataaaaatataattaattatgtccttattaaattgtat

     ttatatacaacatatcatattggctacttaatattaacaataataattacttaataataa
 301 ---------+---------+---------+---------+---------+---------+ 360
     aatatatgttgtatagtataaccgatgaattataattgttattattaatgaattattatt

     attatttcaatagttataaaataaattatttcaattcttatacatttggataaactatta
 361 ---------+---------+---------+---------+---------+---------+ 420
     taataaagttatcaatattttatttaataaagttaagaatatgtaaacctatttgataat

     aaataatgagacatgaacgttcaagttactataaaaataactcaaaataacattattat
 421 ---------+---------+---------+---------+---------+---------+ 480
     tttattactctgtacttgcaagttcaatgatattttattgagtttttattgtaataata

     tgatatgtgtgtgtatgtgtatgtcgttttttctaaatgtcatcaagggtattgatggatg
 481 ---------+---------+---------+---------+---------+---------+ 540
     actatacacacacatacacatacagcaaaaagatttacagtagttcccataactacctac

     tgaatttcatattattatttcagGTGAGAGAACACAGATCAAGAATAGACATATGTACTT
 541 ---------+---------+---------+---------+---------+---------+ 600
     acttaaagtataataataaagtcCACTCTCTTGTGTCTAGTTCTTATCTGTATACATGAA
                             E  R  T  Q  I  K  N  R  H  M  Y  L
```

EP 0 309 862 B1

```
      AACAGAAGAGATACTGAAAGAGAACCCTAACATGTGCGCATATAAGGCACCGTCGTTGGA
601   ---------+---------+---------+---------+---------+---------+  660
      TTGTCTTCTCTATGACTTTCTCTTGGGATTGTACACGCGTATATTCCGTGGCAGCAACCT

       T   E   E   I   L   K   E   N   P   N   M   C   A   Y   K   A   P   S   L   D   -

      TGCAAGAGAAGACATGATGATCAGGGAGGTACCAAGGGTTGGAAAAGAGGCTGCAACCAA
661   ---------+---------+---------+---------+---------+---------+  720
      ACGTTCTCTTCTGTACTACTAGTCCCTCCATGGTTCCCAACCTTTTCTCCGACGTTGGTT

       A   R   E   D   M   M   I   R   E   V   P   R   V   G   K   E   A   A   T   K   -

      GGCCATCAAGGAATGGGGCCAGCCAATGTCTAAGATCACACATTTGATCTTCTGCACCAC
721   ---------+---------+---------+---------+---------+---------+  780
      CCGGTAGTTCCTTACCCCGGTCGGTTACAGATTCTAGTGTGTAAACTAGAAGACGTGGTG

       A   I   K   E   W   G   Q   P   M   S   K   I   T   H   L   I   F   C   T   T   -

      CAGCGGCGTTGCGTTGCCTGGCGTTGATTATGAACTCATCGTACTCTTAGGGCTCGACCC
781   ---------+---------+---------+---------+---------+---------+  840
      GTCGCCGCAACGCAACGGACCGCAACTAATACTTGAGTAGCATGAGAATCCCGAGCTGGG

       S   G   V   A   L   P   G   V   D   Y   E   L   I   V   L   L   G   L   D   P   -

      AAGTGTCAAGAGGTACATGATGTACCACCAAGGTTGCTTTGCTGGTGGCACTGTCCTTCG
841   ---------+---------+---------+---------+---------+---------+  900
      TTCACAGTTCTCCATGTACTACATGGTGGTTCCAACGAAACGACCACCGTGACAGGAAGC

       S   V   K   R   Y   M   M   Y   H   Q   G   C   F   A   G   G   T   V   L   R   -

      TTTGGCTAAGGACTTGGCAGAAAACAACAAGGATGCTCGTGTGCTTATTGTTTGTTCTGA
901   ---------+---------+---------+---------+---------+---------+  960
      AAACCGATTCCTGAACCGTCTTTTGTTGTTCCTACGAGCACACGAATAACAAACAAGACT

       L   A   K   D   L   A   E   N   N   K   D   A   R   V   L   I   V   C   S   E   -

                       P
                       s
                       t
                       I
      GAATACTGCAGTCACTTTTCGTGGTCCTAATGAGACAGACATGGATAGTCTTGTAGGGCA
961   ---------+---------+---------+---------+---------+---------+  1020
      CTTATGACGTCAGTGAAAAGCACCAGGATTACTCTGTCTGTACCTATCAGAACATCCCGT

       N   T   A   V   T   F   R   G   P   N   E   T   D   M   D   S   L   V   G   Q   -

      AGCATTGTTTGCCGATGGAGCTGCTGCAATTATCATTGGTTCTGATCCTGTTCCAGAGGT
1021  ---------+---------+---------+---------+---------+---------+  1080
      TCGTAACAAACGGCTACCTCGACGACGTTAATAGTAACCAAGACTAGGACAAGGTCTCCA

       A   L   F   A   D   G   A   A   A   I   I   I   G   S   D   P   V   P   E   V   -

      TGAGAATCCTCTCTTTGAGATTGTTTCAACTGATCAACAACTTGTCCCTAACAGCCATGG
1081  ---------+---------+---------+---------+---------+---------+  1140
      ACTCTTAGGAGAGAAACTCTAACAAAGTTGACTAGTTGTTGAACAGGGATTGTCGGTACC

       E   N   P   L   F   E   I   V   S   T   D   Q   Q   L   V   P   N   S   H   G   -

      AGCCATCGGTGGTCTCCTTCGTGAAGTTGGACTTACATTTTATCTTAACAAGAGTGTTCC
1141  ---------+---------+---------+---------+---------+---------+  1200
      TCGGTAGCCACCAGAGGAAGCACTTCAACCTGAATGTAAAATAGAATTGTTCTCACAAGG

       A   I   G   G   L   L   R   E   V   G   L   T   F   Y   L   N   K   S   V   P   -
```

18

```
                                                    H
                                                    i
                                                    n
                                                    d
                                                    I
                                                    I
                                                    I

      GGATATTATTTCACAAAACATCAATGGTGCACTCAGTAAAGCTTTTGATCCACTGGGTAT
1201  ---------+---------+---------+---------+---------+---------+  1260
      CCTATAATAAAGTGTTTTGTAGTTACCACGTGAGTCATTTCGAAAACTAGGTGACCCATA

       D   I   I   S   Q   N   I   N   G   A   L   S   K   A   F   D   P   L   G   I  -

      ATCTGATTATAACTCAATATTTTGGATTGCACATCTTGGTGGACGCGCAATTTTGGACCA
1261  ---------+---------+---------+---------+---------+---------+  1320
      TAGACTAATATTGAGTTATAAAACCTAACGTGTAGAACCACCTGCGCGTTAAAACCTGGT

       S   D   Y   N   S   I   F   W   I   A   H   L   G   G   R   A   I   L   D   Q  -

      AGTTGAACAGAAGGTGAACTTGAAGCCAGAGAAGATGAAAGCCACTAGAGATGTACTTAG
1321  ---------+---------+---------+---------+---------+---------+  1380
      TCAACTTGTCTTCCACTTGAACTTCGGTCTCTTCTACTTTCGGTGATCTCTACATGAATC

       V   E   Q   K   V   N   L   K   P   E   K   M   K   A   T   R   D   V   L   S  -

      CAATTATGGTAACATGTCAAGTGCGTGTGTGTTCTTCATTATGGATTTGATGAGAAAGAA
1381  ---------+---------+---------+---------+---------+---------+  1440
      GTTAATACCATTGTACAGTTCACGCACACACAAGAAGTAATACCTAAACTACTCTTTCTT

       N   Y   G   N   M   S   S   A   C   V   F   F   I   M   D   L   M   R   K   K  -

      GTCACTTGAAACTGGACTTAAAACCACTGGAGAAGGACTTGATTGGGGTGTGTTGTTTGG
1441  ---------+---------+---------+---------+---------+---------+  1500
      CAGTGAACTTTGACCTGAATTTTGGTGACCTCTTCCTGAACTAACCCCACACAACAAACC

       S   L   E   T   G   L   K   T   T   G   E   G   L   D   W   G   V   L   F   G  -

      TTTTGGCCCTGGTCTCACTATTGAAACCGTTGTTCTCCGCAGCATGGCCATAtaatacgc
1501  ---------+---------+---------+---------+---------+---------+  1560
      AAAACCGGGACCAGAGTGATAACTTTGGCAACAAGAGGCGTCGTACCGGTATattatgcg

       F   G   P   G   L   T   I   E   T   V   V   L   R   S   M   A   I   *

      ttaattatatatctctgcatatatgcaattttgttattttttaataattttcttttactc
1561  ---------+---------+---------+---------+---------+---------+  1620
      aattaatatatagagacgtatatacgttaaaacaataaaaaattattaaaagaaaatgag

      taaaataagattctaaatggcttatattcttagatgagtgaaaacttagacagagatgtc
1621  ---------+---------+---------+---------+---------+---------+  1680
      attttattctaagatttaccgaatataagaatctactcacttttgaatctgtctctacag

      taaagttaattcgttatgcgaaga
1681  ---------+---------+----  1704
      atttcaattaagcaatacgcttct
```

Im folgenden werden einige der bei der Transformation von Pflanzen bzw. Pflanzenzellen eingesetzte Medien beschrieben:

Am-Medium

19

| 3,5 g | K$_2$HPO$_4$ |
|-------|--------------|
| 1,5 g | KH$_2$PO$_4$ |
| 0,5 g | Na$_3$ Citrat |
| 0,1 g | MgSO$_4$ x 7H$_2$O |
| 1 g | (NH$_4$)$_2$SO$_4$ |
| 2 g | Glukose |
| | ad 1 l |

Medium für sterile Sproßkultur von Tabak

Macro-elemente 1/2 der Konzentration der MS Salze

Micro-elemente 1/2 der Konzentration der MS Salze

Fe-EDTA           Murashige und Skoog (MS)

| Myo-Inosit | | 100 | mg/l |
|------------|--|-----|------|
| Sucrose | | 10 | mg/l |
| Agar | | 8 | g/l |
| Vitamine | Ca-panthotenat | 1 | mg/l |
| | Biotin | 10 | mg/l |
| | Nicotinsäure | 1 | mg/l |
| | Pyridoxin | 1 | mg/l |
| | Thiamin | 1 | mg/l |

pH 5,7 vor dem Autoklavieren

K3-Medium

Zur Kultur von Nicotiana tabacum petit Havana SR1, Nicotiana tabacum Wisconsin 38, und Nicotiana plumaginifolia Protoplasten (Nagy und Maliga, 1976)

| Macro-elemente | $NH_4NO_3$ | 250 mg/l |
| | $KNO_3$ | 2500 mg/l |
| | $CaCl_2 \bullet 2H_2O$ | 900 mg/l |
| | $MgSO_4.7H_2O$ | 250 mg/l |
| | $NaH_2PO_4.1H_2O$ | 150 mg/l |
| | $(NH_4)_2SO_4$ | 134 mg/l |
| | $CaHPO_4.1H_2O$ | 50 mg/l |
| Micro-elemente | $H_3BO_3$ | 3 mg/l |
| | $MnSO_4.1H_2O$ | 10 mg/l |
| | $ZnSO_4.4H_2O$ | 2 mg/l |
| | $KI$ | 0,75 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Sucrose | | 137 g/l ( = 0,4 M) |
| Xylose | | 250 mg/l |
| Vitamine | Nicotinsäure | 1 mg/l |
| | Pyridoxin | 1 mg/l |
| | Thiamin | 10 mg/l |
| Hormone | NAA | 1,0 mg/l |
| | Kinetin | 0,2 mg/l |
| pH 5,6<br>Filter sterilisieren | | |

Linsemaier und Skoog Medium (Linsemaier und Skoog 1965)

Zur Kultur von regenerierenden Protoplasten und für Gewebekultur von Tabaktumoren und Kallus. Linsemaier und Skoog (LS) Medium ist Murashige und Skoog Medium (Murashige und Skoog, 1962) mit den folgenden Modifikationen:
- Thiamin wird in höherer Konzentration eingewogen 0,4 mg/l anstatt 0,1 mg/l;
- Glycin, Pyridoxin und Nicotinsäure fehlen.

| Macro-elemente | $NH_4NO_3$ | 1650 mg/l |
| | $KNO_3$ | 1900 mg/l |
| | $CaCl_2.2H_2O$ | 440 mg/l |
| | $MgSO_4.7H_2O$ | 370 mg/l |
| | $KH_2PO_4$ | 170 mg/l |
| Micro-elemente | $H_3BO_3$ | 6,2 mg/l |
| | $MnSO_4.1H_2O$ | 22,3 mg/l |
| | $ZnSO_4.4H_2O$ | 8,6 mg/l |
| | KI | 0,83 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | Thiamin | 100 mg/l |
| Saccharose | | 30 g/l |
| Agar | | 8 g/l |
| Vitamine | | 0,4 mg/l |
| Hormone: | NAA | 1 mg/l |
| | Kinetin | 0,2 mg/l |
| pH 5,7 vor dem Autoklavieren | | |

Zur Transformation von Pflanzen bzw. Pflanzenzellen kann die folgende Literatur angeführt weren:

Aerts M, Jacobs M, Hernalsteens JP, Van Montagu M, Schell J (1983) Induction and in vitro culture of Arabidopsis thaliana crown gall tumours, Plant Sci Lett. 17: 43-50

Atamasov A, Brown DCW (1984) Plant regeneration from suspension culture and mesophyll protoplasts of Medicago sativa L. Plant Cell Tiss Org. Cult. 3, 149-162

Czernilofsky et al. (1986) Studies of the Structure and Functional Organization of Foreign DNA Integrates into the Genome of Nicotiana tabacum. DNA, Vol. 5, No. 6 (1986), 473

Davey MR, Cocking EC, Freeman J, Pearce N, Tudor I (1980) Transformation of Petunia protoplasts by isolated Agrobacterium plasmid. Plant Sci Lett 18: 307-313

Deblaere R., Bytebier B., De Greve H, Deboeck F, Schell J, van Montagu M, Leemans J (1985) Efficient octopine Ti plasmid-derived vectors for Agrobacterium-mediated gene transfer to plants. Nucleic Acid Research, Vol. 13, No. 13, 4777 (1985)

Fromm ME, Taylor LP, Walbot V (1986) Stable transformation of maize after gene transfer by electroporation. Nature 319: 791-793

Hain, R., Stabel, P., Czernilofsky, A.Pp., Steinbiß, H.H., Herrera-Estrella, L., Schell, J. (1985) Uptake, integration, expression and genetic transmission of a selectable chimeric gene by plant protoplasts. Molec Gen Genet 199: 161-168

Hernalsteens JP, Thia-Tong L, Schell J, Van Montagu M (1984) An Agrobacterium-transformed Cell culture from the monocot Asparagus officinalis. EMBO J 3:3039-3041

Herrera-Estrella L., De Block M., Messens E., Hernalsteens JP., van Montagu M., Schell J. (1983) EMBO J. 2: 987-995.

Hooykaas-Van Slogteren GMS, Hooykaas PJJ, Schilperoort RA (1984) Expression of Ti-plasmid genes in monocotyledonous plants infected with Agrobacterium tumefaciens Nature 311: 763-764

Horsch RB, Fry JE, Hoffmann NL, Eichholtz D, Rogers SG, Fraley RT (1985) A simple and general method for transferring genes into plants. Science 277: 1229-1231

Kao KN, Michayluk MR (1980) Plant regeneration from Mesophyll protoplasts of Alfalfa. Z Pflanzenphysiol. 96, 135-141

Keller WA, Melchers G (1973) The effect of high pH and calcium on tobacco leaf protoplast fusion. Z Naturforschg 28c: 737-741

Krens FH, Molendijk L, Wullems GJ, Schilperoort RA (1982) in vitro transformation of plant protoplasts with Ti-plasmid DNA. Nature 296: 72-74

Koncz C, Schell J (1986) The promotor of $T_L$-DNA gene 5 controls the tissue-specific expression of chimaeric genes carried by a noval type of Agrobacterium linary vector. Mol. Gen. Genet. (1986) 204: 338-396
Linsmaier DM, Skoog F (1965) Organic growth factor requirements of tobacco tissue cultures. Physiol Plant 18: 100-127
Marton L, Wullems GJ, Molendijk L, Schilperoort PR (1979) In vitro transformation of cultured cells from Nicotiana tabacum by Agrobacterium tumefaciens. Nature 277: 1229-131
Nagy JI, Maliga P (1976) Callus induction and plant regeneration from mesophyll protoplasts of Nicotiana sylvestris. Z. Pflanzenphysiol 78: 453-455
Otten LABM, Schilperoort RA (1978) A rapid microscale method for the detection of Lysopin and Nopalin dehydrogenase activities. Biochim biophys acta 527: 497-500
Paszkowski J, Shillito RD, Saul M, Mandak V, Hohn T, Hohn B, Potrykus I (1984) Direct gene transfer to plants. EMBO J 3: 2717-2722
Potrykus I, Saul MW, Petruska J, Paszkowski J, Shillito RD (1985) Direct gene transfer to cells of a gramineous monocot. Molec gen genet 199: 183-188
Shenk RU, Hildebrandt AC (1972) Medium and techniques for induction and growth of monocotyledonous and dicotyledonous cell cultures. Ca. J. Bot. 50, 199-204
Shillito RD, Paszkowski J. Potrykus I (1983) Agarose plating and Bead type culture technique enable and stimulate development of protoplast-derived colonies in an number of plant species. Pl Cell Rep 2: 244-247
Simons-Schreier A, Dissertation Universität Köln (1988) Studien zur Transformation von Medicago sativa und zur Expression eines Leghämoglobingens (1bc3) und eines chimären 1b-cat-Gens während der Symbiose in transgenen Pflanzen
Stuart DA, Strickland SG (1984) Somatic embryogenesis from cell cultures of Medicago sativa L. I. The role of amino acid additions to the regeneration medium. Plant Sci. Let. 34, 165-174
Stuart DA, Strickland SG (1984) Somatic embryogenesis from cell cultures of Medicago sativa L. II. The interaction of amino acids with ammonium. Plant Sci. Let. 34, 175-181
Van den Elzen PJM, Townsend J, Lee KY, Bedbrook JR (1985) Achimaeric resistance gen as a selectable marker in plant cells. Plant Mol. Biol. 5, 299-302.
Velten J, Velten L, Hain R, Schell J (1984) Isolation of a dual plant promotor fragment from the Ti Plasmid of Agrobacterium tumefaciens. EMBO J 12: 2723-2730
Walker KA, Yu PC, Sato SJ, Jaworski EG (1978) The hormonal control of organ formation in callus of Medicago sativa L. cultured in vitro. Amer. J. Bot. 65(6), 654-659
Walker KA, Sato SJ (1981) Morphogenesis in callus tissue of Medicago sativa: The role of ammonium ion in somatic ambryogenesis. Plant Cell Tiss. Org. Cult. 1, 109-121
Wullems GJ, Molendijk L, Ooms G, Schilperoort RA (1981) Differential expression of crown gall tumor markers in transformants obtained after in vitro Agrobacterium tumefaciens - induced transformation of cell wall regenerating protoplasts derived from Nicotiana tabacum. Proc Natl Acad Sci 78: 4344-4348
Zambryski P, Joos H, Genetello C, van Montagu M, Schell J (1983) Ti-plasmid vector for the introduction of DNA into plant cells without altering their normal regeneration capacity, EMBO J 12: 2143-2150.
Bernd Reiss, Rolf Sprengel, Hans Will and Heinz Schaller (1984) A new sensitive method for qualitative and quantitative assay of neomycin phosphotransferase in crude cell tracts, GENE 1081: 211-217
Peter H. Schreier, Elisabeth A. Seftor, Jozef Schell and Hans. J. Bohnert (1985) The use of nuclear-encoded sequences to direct the light-regulated synthesis and transport of a foreign protein into plant chloroplasts, EMBO J Vol. 4, No. 1: 25-32
Gamborg O. L., Miller R. A. and Ojima K. (1968) Nutrient requirements of suspension cultures of soybean root cells, Experimental Cell Research 50: 151-158
Michael M. Oelck, Dissertation, 1984, University of Cologne, Federal Republic of Germany, Regeneration von Leguminosen aus Gewebe- und Zellkulturen
Weiterhin können die folgenden veröffentlichten Patentanmeldungen aufgeführt werden:
EP-A 116 718
EP-A 159 418
EP-A 120 515
EP-A-120 516
EP-A-172 112
EP-A-140 556
EP-A-174 166
EP-A-122 791
EP-A-126 546
EP-A-164 597
EP-A-175 966

EP 0 309 862 B1

WO 84/02913
WO 84/02919
WO 84/02920
WO 83/01176

Erläuterungen zu Fig. 1 und Fig. 2

Fig. 1 stellt den durch partielle Spaltung mit EcoRI und HindIII aus dem Plasmid pGS 828.1 erhaltenen Nukleinsäureabschnitt mit 6700 Nukleotid-Paaren dar, der das Stilbensynthase-Gen enthält.

(1) und (2) deuten Anfang (1) und Ende (2) des Strukturgens an. Der Regulator-Teil des Gens befindet sich auf dem linken Abschnitt (links von (1)). Folgende Restriktionsenzymschnittstellen sind angegeben:

E: EcoRI
H: HindIII
P: PstI
S: SstI

Fig. 2 stellt die Gen-Einheit gemäß Fig. 1 im Vektor pSP 65 dar. Das gesamte Plasmid trägt die Bezeichnung pGS828.1.

"ORI" bedeutet Origin of Replication (die Sequenzen im Vektor pSP65, die für die Vermehrung des Plasmids in Escherichia coli wichtig sind). "AMP$^R$" steht für das Gen welche die Resistenz von pSP65 enthaltenden Escherichia coli gegen Ampicillin bewirkt. Außer den in Fig. 1 angegebenen Restriktionsschnittstellen ist die als Pv bezeichnete PvuII Schnittstelle angegeben.

**Patentansprüche**

1. Stilbensynthase-Gen, entsprechend dem Stilbensynthase-Gen, welches im Plasmid pGS 828.1 (erhältlich aus E. coli DSM 4243) enthalten ist, sowie die davon abgeleiteten DNA-Sequenzen mit Stilbensynthase-Aktivität.

2. Stilbensynthase-Gen gemäß Anspruch 1, wobei die Stilbensynthase Resveratrolsynthase und Pinosylvinsynthase bedeutet.

3. Stilbensynthase-Gen gemäß den Ansprüchen 1 und 2, erhältlich aus Erdnuß (Arachis hypogaea), Pinus und Wein.

4. Stilben-Synthase Gen-Einheit, welche aus etwa 6700 Basenpaaren besteht und 3 EcoRI, 3 HindIII und 1 PstI Schnittstellen aufweist und durch eine partielle Spaltung des Plasmids pGS 828.1 (erhältlich aus E. coli DSM 4243) mit EcoRI und HindIII oder aus dem Plasmid pGS 828.1 (erhältlich aus E. coli DSM 4243) mit Hilfe von SstI und PvuII erhältlich ist.

5. Regulatorisch wirkender Teil des StilbensynthaseGens gemäß den Ansprüchen 1 bis 4.

6. Struktur-Gen des Stilbensynthase-Gens gemäß den Ansprüchen 1 bis 4.

7. DNA mit folgender Sequenz, welche sowohl mit oder ohne Intron vorliegen kann sowie die davon abgeleiteten DNA-Sequenzen mit Stilbensynthase Aktivität:

24

```
                                            E
                                            c
                                            o
                                            R
                                            I
     cacagctaagaaaagATGGTGTCTGTGAGTGGAATTCGCAAGGTTCAAAGGGCAGAAGGT
  1  ---------+---------+---------+---------+---------+---------+  60
     gtgtcgattcttttcTACCACAGACACTCACCTTAAGCGTTCCAAGTTTCCCGTCTTCCA

              M   V   S   V   S   G   I   R   K   V   Q   R   A   E   G   -

     CCAGCAACGGTATTGGCGATCGGAACAGCAAATCCACCAAACTGTGTTGATCAGAGTACA
 61  ---------+---------+---------+---------+---------+---------+ 120
     GGTCGTTGCCATAACCGCTAGCCTTGTCGTTTAGGTGGTTTGACACAACTAGTCTCATGT

       P   A   T   V   L   A   I   G   T   A   N   P   P   N   C   V   D   Q   S   T   -

     TATGCAGATTATTATTTTAGAGTAACCAACGGCGAACACATGACTGATCTCAAGAAGAAA
121  ---------+---------+---------+---------+---------+---------+ 180
     ATACGTCTAATAATAAAATCTCATTGGTTGCCGCTTGTGTACTGACTAGAGTTCTTCTTT

       Y   A   D   Y   Y   F   R   V   T   N   G   E   H   M   T   D   L   K   K   K   -

     TTTCAGCGCATCTgtatgtattttttattaagcgttctatatttgtttatatttaatattt
181  ---------+---------+---------+---------+---------+---------+ 240
     AAAGTCGCGTAGAcatacataaaaataattcgcaagatataaacaaatataaattataaa

       F   Q   R   I   C

     atcaaaataaaattcgttctttttatttttatattaattaatacaggaataatttaacata
241  ---------+---------+---------+---------+---------+---------+ 300
     tagttttatttttaagcaagaaaataaaaatataattaattatgtccttattaaattgtat

     ttatatacaacatatcatattggctacttaatattaacaataataattacttaataataa
301  ---------+---------+---------+---------+---------+---------+ 360
     aatatatgttgtatagtataaccgatgaattataattgttattattaatgaattattatt

     attatttcaatagttataaaataaattatttcaattcttatacatttggataaactatta
361  ---------+---------+---------+---------+---------+---------+ 420
     taataaagttatcaatattttatttaataaagttaagaatatgtaaacctatttgataat

     aaataatgagacatgaacgttcaagttactataaaaataactcaaaaataacattattat
421  ---------+---------+---------+---------+---------+---------+ 480
     tttattactctgtacttgcaagttcaatgatattttttattgagttttttattgtaataata

     tgatatgtgtgtgtatgtgtatgtcgtttttctaaatgtcatcaagggtattgatggatg
481  ---------+---------+---------+---------+---------+---------+ 540
     actatacacacacatacacatacagcaaaaagatttacagtagttcccataactacctac

     tgaatttcatattattatttcagGTGAGAGAACACAGATCAAGAATAGACATATGTACTT
541  ---------+---------+---------+---------+---------+---------+ 600
     acttaaagtataataataaagtcCACTCTCTTGTGTCTAGTTCTTATCTGTATACATGAA
                            E   R   T   Q   I   K   N   R   H   M   Y   L
```

EP 0 309 862 B1

```
     AACAGAAGAGATACTGAAAGAGAACCCTAACATGTGCGCATATAAGGCACCGTCGTTGGA
 601 ---------+---------+---------+---------+---------+---------+ 660
     TTGTCTTCTCTATGACTTTCTCTTGGGATTGTACACGCGTATATTCCGTGGCAGCAACCT

      T  E  E  I  L  K  E  N  P  N  M  C  A  Y  K  A  P  S  L  D  -

     TGCAAGAGAAGACATGATGATCAGGGAGGTACCAAGGGTTGGAAAAGAGGCTGCAACCAA
 661 ---------+---------+---------+---------+---------+---------+ 720
     ACGTTCTCTTCTGTACTACTAGTCCCTCCATGGTTCCCAACCTTTTCTCCGACGTTGGTT

      A  R  E  D  M  M  I  R  E  V  P  R  V  G  K  E  A  A  T  K  -

     GGCCATCAAGGAATGGGGCCAGCCAATGTCTAAGATCACACATTTGATCTTCTGCACCAC
 721 ---------+---------+---------+---------+---------+---------+ 780
     CCGGTAGTTCCTTACCCCGGTCGGTTACAGATTCTAGTGTGTAAACTAGAAGACGTGGTG

      A  I  K  E  W  G  Q  P  M  S  K  I  T  H  L  I  F  C  T  T  -

     CAGCGGCGTTGCGTTGCCTGGCGTTGATTATGAACTCATCGTACTCTTAGGGCTCGACCC
 781 ---------+---------+---------+---------+---------+---------+ 840
     GTCGCCGCAACGCAACGGACCGCAACTAATACTTGAGTAGCATGAGAATCCCGAGCTGGG

      S  G  V  A  L  P  G  V  D  Y  E  L  I  V  L  L  G  L  D  P  -

     AAGTGTCAAGAGGTACATGATGTACCACCAAGGTTGCTTTGCTGGTGGCACTGTCCTTCG
 841 ---------+---------+---------+---------+---------+---------+ 900
     TTCACAGTTCTCCATGTACTACATGGTGGTTCCAACGAAACGACCACCGTGACAGGAAGC

      S  V  K  R  Y  M  M  Y  H  Q  G  C  F  A  G  G  T  V  L  R  -

     TTTGGCTAAGGACTTGGCAGAAAACAACAAGGATGCTCGTGTGCTTATTGTTTGTTCTGA
 901 ---------+---------+---------+---------+---------+---------+ 960
     AAACCGATTCCTGAACCGTCTTTTGTTGTTCCTACGAGCACACGAATAACAAACAAGACT

      L  A  K  D  L  A  E  N  N  K  D  A  R  V  L  I  V  C  S  E  -
                     P
                     s
                     t
                     I
     GAATACTGCAGTCACTTTTCGTGGTCCTAATGAGACAGACATGGATAGTCTTGTAGGGCA
 961 ---------+---------+---------+---------+---------+---------+ 1020
     CTTATGACGTCAGTGAAAAGCACCAGGATTACTCTGTCTGTACCTATCAGAACATCCCGT

      N  T  A  V  T  F  R  G  P  N  E  T  D  M  D  S  L  V  G  Q  -

     AGCATTGTTTGCCGATGGAGCTGCTGCAATTATCATTGGTTCTGATCCTGTTCCAGAGGT
1021 ---------+---------+---------+---------+---------+---------+ 1080
     TCGTAACAAACGGCTACCTCGACGACGTTAATAGTAACCAAGACTAGGACAAGGTCTCCA

      A  L  F  A  D  G  A  A  A  I  I  I  G  S  D  P  V  P  E  V  -

     TGAGAATCCTCTCTTTGAGATTGTTTCAACTGATCAACAACTTGTCCCTAACAGCCATGG
1081 ---------+---------+---------+---------+---------+---------+ 1140
     ACTCTTAGGAGAGAAACTCTAACAAAGTTGACTAGTTGTTGAACAGGGATTGTCGGTACC

      E  N  P  L  F  E  I  V  S  T  D  Q  Q  L  V  P  N  S  H  G  -

     AGCCATCGGTGGTCTCCTTCGTGAAGTTGGACTTACATTTTATCTTAACAAGAGTGTTCC
1141 ---------+---------+---------+---------+---------+---------+ 1200
     TCGGTAGCCACCAGAGGAAGCACTTCAACCTGAATGTAAAATAGAATTGTTCTCACAAGG

      A  I  G  G  L  L  R  E  V  G  L  T  F  Y  L  N  K  S  V  P  -
```

26

```
                                              H
                                              i
                                              n
                                              d
                                              I
                                              I
                                              I
     GGATATTATTTCACAAAACATCAATGGTGCACTCAGTAAAGCTTTTGATCCACTGGGTAT
1201 ---------+---------+---------+---------+---------+---------+ 1260
     CCTATAATAAAGTGTTTTGTAGTTACCACGTGAGTCATTTCGAAAACTAGGTGACCCATA

      D   I   I   S   Q   N   I   N   G   A   L   S   K   A   F   D   P   L   G   I   -

     ATCTGATTATAACTCAATATTTTGGATTGCACATCTTGGTGGACGCGCAATTTTGGACCA
1261 ---------+---------+---------+---------+---------+---------+ 1320
     TAGACTAATATTGAGTTATAAAACCTAACGTGTAGAACCACCTGCGCGTTAAAACCTGGT

      S   D   Y   N   S   I   F   W   I   A   H   L   G   G   R   A   I   L   D   Q   -

     AGTTGAACAGAAGGTGAACTTGAAGCCAGAGAAGATGAAAGCCACTAGAGATGTACTTAG
1321 ---------+---------+---------+---------+---------+---------+ 1380
     TCAACTTGTCTTCCACTTGAACTTCGGTCTCTTCTACTTTCGGTGATCTCTACATGAATC

      V   E   Q   K   V   N   L   K   P   E   K   M   K   A   T   R   D   V   L   S   -

     CAATTATGGTAACATGTCAAGTGCGTGTGTGTTCTTCATTATGGATTTGATGAGAAAGAA
1381 ---------+---------+---------+---------+---------+---------+ 1440
     GTTAATACCATTGTACAGTTCACGCACACACAAGAAGTAATACCTAAACTACTCTTTCTT

      N   Y   G   N   M   S   S   A   C   V   F   F   I   M   D   L   M   R   K   K   -

     GTCACTTGAAACTGGACTTAAAACCACTGGAGAAGGACTTGATTGGGGTGTGTTGTTTGG
1441 ---------+---------+---------+---------+---------+---------+ 1500
     CAGTGAACTTTGACCTGAATTTTGGTGACCTCTTCCTGAACTAACCCCACACAACAAACC

      S   L   E   T   G   L   K   T   T   G   E   G   L   D   W   G   V   L   F   G   -

     TTTTGGCCCTGGTCTCACTATTGAAACCGTTGTTCTCCGCAGCATGGCCATAtaatacgc
1501 ---------+---------+---------+---------+---------+---------+ 1560
     AAAACCGGGACCAGAGTGATAACTTTGGCAACAAGAGGCGTCGTACCGGTATattatgcg

      F   G   P   G   L   T   I   E   T   V   V   L   R   S   M   A   I   *

     ttaattatatatctctgcatatatgcaattttgttattttttaataattttcttttactc
1561 ---------+---------+---------+---------+---------+---------+ 1620
     aattaatatatagagacgtatatacgttaaaacaataaaaaattattaaaagaaaatgag

     taaaataagattctaaatggcttatattcttagatgagtgaaaacttagacagagatgtc
1621 ---------+---------+---------+---------+---------+---------+ 1680
     attttattctaagatttaccgaatataagaatctactcacttttgaatctgtctctacag

     taaagttaattcgttatgcgaaga
1681 ---------+---------+---- 1704
     atttcaattaagcaatacgcttct
```

**8.** DNA bestehend aus der oder rekombinante DNA enthaltend die DNA-Sequenz der codierenden Region der DNA gemäß Anspruch 7, einschließlich der davon abgeleiteten DNA-Sequenzen mit Stilbensynthase Aktivität,

**9.** Stilbensynthase-Gen- bzw. Gen-Einheit enthaltend die DNA gemäß Anspruch 7.

**10.** Rekombinante prokaryontische oder eukaryontische DNA, welche ein oder mehrere Stilbensynthase-Gene bzw. Gen-Einheiten und/oder deren Strukturgene oder deren regulatorischen Genteile und/oder die DNA gemäß den Ansprüchen 1 bis 9 als "fremde" DNA oder als "zusätzliche" DNA enthält.

**11.** Rekombinante DNA gemäß Anspruch 10, welche in Pflanzenzellen (einschließlich Protoplasten) oder Pflanzen (einschließlich Pflanzenteilen und Samen) enthalten ist.

**12.** Vektoren, welche ein oder mehrere Stilbensynthase-Gene bzw. Gen-Einheiten oder deren Strukturgene oder deren regulatorischen Genteile und/oder die DNA gemäß den Ansprüchen 1 bis 9 und/oder die rekombinante DNA gemäß Anspruch 10 enthalten.

**13.** Vektor-Plasmid pGS 828.1 (erhältlich aus E. coli DSM 4243).

**14.** Transformierte Mikroorganismen, welche ein oder mehrere Stilbensynthase-Gene bzw. Gen-Einheiten und/oder deren Strukturgene oder deren regulatorischen Genteile und/oder die DNA gemäß den Ansprüchen 1 bis 9 und/oder die rekombinante DNA gemäß Anspruch 10 enthalten.

**15.** Escherichia coli Stamm Nurdug 2010 (entsprechend E. coli DSM 4243) sowie seine Mutanten und Varianten.

**16.** Verwendung des Stilbensynthase-Gens bzw. der Gen-Einheit und/oder dessen Strukturgens oder dessen regulatorischen Genteiles und/oder der DNA und/oder der rekombinanten DNA und/oder der Vektoren und/oder der transformierten Mikroorganismen gemäß den Ansprüchen 1 bis 15 zur Transformation von Pflanzenzellen (einschließlich Protoplasten) und Pflanzen (einschließlich Pflanzenteilen und Samen).

**17.** Transformierte Pflanzenzellen (einschließlich Protoplasten) und Pflanzen (einschließlich Pflanzenteilen und Samen), welche ein oder mehrere Stilbensynthase-Gene bzw. Gen-Einheiten und/oder deren Strukturgene oder deren regulatorischen Genteile und/oder die DNA und/oder die rekombinante DNA gemäß den Ansprüchen 1 bis 10 als "fremde" oder "zusätzliche" DNA enthalten.

**18.** Verfahren zur Herstellung transformierter Pflanzenzellen (einschließlich Protoplasten) und Pflanzen (einschließlich Pflanzenteile und Samen) gemäß Anspruch 17, dadurch gekennzeichnet, daß man
(a) ein oder mehrere Stilbensynthase-Gene bzw. Gen-Einheiten und/oder deren Strukturgene oder deren regulatorischen Genteile und/oder die DNA und/oder die rekombinante DNA gemäß den Ansprüchen 1 bis 10 in den Genom von Pflanzenzellen (einschließlich Protoplasten) einsetzt und gegebenenfalls
(b) aus den transformierten Pflanzenzellen (einschließlich Protoplasten) vollständige transformierte Pflanzen regeneriert und gegebenenfalls
(c) von den so erhaltenen transformierten Pflanzen die gewünschten Pflanzenteile (einschließlich Samen) gewinnt.

**19.** Verwendung von transformierten Pflanzenzellen (einschließlich Protoplasten) gemäß Anspruch 17 zur Erzeugung von Vermehrungsmaterial sowie zur Erzeugung neuer Pflanzen und deren Vermehrungsmaterial.

**20.** Vermehrungsmaterial, erhältlich durch die Vermehrung der transformierten Pflanzenzellen und Pflanzen gemäß Anspruch 17.

**21.** Transformierte Pflanzenzellen (einschließlich Protoplasten) und Pflanzen (einschließlich Pflanzenteile und Samen) gemäß Anspruch 17, das Verfahren zu ihrer Herstellung gemäß Anspruch 18 und ihre Verwendung gemäß Anspruch 19 sowie das Vermehrungsmaterial gemäß Anspruch 20, wobei die Pflanzenzellen und Pflanzen Tabak-, Luzerne- oder Kartoffelpflanzenzellen und Tabak-, Luzerne- oder Kartoffelpflanzen sind.

**Claims**

**1.** Stilbene synthase gene corresponding to the stilbene synthase gene which is contained in plasmid pGS 828.1 (obtainable from E. coli DSM 4243), and the DNA sequences derived therefrom with stilbene synthase activity.

**2.** Stilbene synthase gene according to Claim 1, in which stilbene stynhase means resveratrol synthase

and pinosylvine synthase.

3. Stilbene synthase gene according to Claims 1 and 2, which can be obtained from groundnut (Arachis hypogaea), Pinus and vine.

4. Stilbene synthase gene unit which consists of approximately 6,700 base pairs and exhibits 3 EcoRI, 3 HindIII and 1 PstI cleavage sites, and which can be obtained by partial cleavage of the plasmid pGS 828.1 (obtainable from E. coli DSM 4243) using EcoRI and HindIII, or from plasmid pGS 828.1 (obtainable from E. coli DSM 4243) with the aid of SstI and PvuII.

5. Part, with regulatory activity, of the stilbene synthase gene according to Claims 1 to 4.

6. Structural gene of the stilbene synthase gene according to Claims 1 to 4.

7. DNA containing the following sequence, which can be present either with or without intron, and the DNA sequences derived therefrom with stilbene synthase activity:

EP 0 309 862 B1

```
                                          E
                                          c
                                          o
                                          R
                                          I
      cacagctaagaaaagATGGTGTCTGTGAGTGGAATTCGCAAGGTTCAAAGGGCAGAAGGT
    1 --------+---------+---------+---------+---------+---------+ 60
      gtgtcgattcttttcTACCACAGACACTCACCTTAAGCGTTCCAAGTTTCCCGTCTTCCA

                    M  V  S  V  S  G  I  R  K  V  Q  R  A  E  G  -

      CCAGCAACGGTATTGGCGATCGGAACAGCAAATCCACCAAACTGTGTTGATCAGAGTACA
   61 --------+---------+---------+---------+---------+---------+ 120
      GGTCGTTGCCATAACCGCTAGCCTTGTCGTTTAGGTGGTTTGACACAACTAGTCTCATGT

      P  A  T  V  L  A  I  G  T  A  N  P  P  N  C  V  D  Q  S  T  -

      TATGCAGATTATTATTTTAGAGTAACCAACGGCGAACACATGACTGATCTCAAGAAGAAA
  121 --------+---------+---------+---------+---------+---------+ 180
      ATACGTCTAATAATAAAATCTCATTGGTTGCCGCTTGTGTACTGACTAGAGTTCTTCTTT

      Y  A  D  Y  Y  F  R  V  T  N  G  E  H  M  T  D  L  K  K  K  -

      TTTCAGCGCATCTgtatgtatttttattaagcgttctatatttgtttatatttaatattt
  181 --------+---------+---------+---------+---------+---------+ 240
      AAAGTCGCGTAGAcatacataaaaataattcgcaagatataaacaaatataaattataaa

      F  Q  R  I  C

      atcaaaataaaattcgttcttttattttttatattaattaatacaggaataatttaacata
  241 --------+---------+---------+---------+---------+---------+ 300
      tagttttatttttaagcaagaaaataaaaatataattaattatgtccttattaaattgtat

      ttatatacaacatatcatattggctacttaatattaacaataataattacttaataataa
  301 --------+---------+---------+---------+---------+---------+ 360
      aatatatgttgtatagtataaccgatgaattataattgttattattaatgaattattatt

      attatttcaatagttataaaataaattatttcaattcttatacatttggataaactatta
  361 --------+---------+---------+---------+---------+---------+ 420
      taataaagttatcaatattttatttaataaagttaagaatatgtaaacctatttgataat

      aaataatgagacatgaacgttcaagttactataaaaataactcaaaataacattattat
  421 --------+---------+---------+---------+---------+---------+ 480
      tttattactctgtacttgcaagttcaatgatattttattgagttttattgtaataata

      tgatatgtgtgtgtatgtgtatgtcgttttttctaaatgtcatcaagggtattcatggatg
  481 --------+---------+---------+---------+---------+---------+ 540
      actatacacacacatacacatacagcaaaaagatttacagtagttcccataactacctac

      tgaatttcatattattatttcagGTGAGAGAACACAGATCAAGAATAGACATATGTACTT
  541 --------+---------+---------+---------+---------+---------+ 600
      acttaaagtataataataaagtcCACTCTCTTGTGTCTAGTTCTTATCTGTATACATGAA

                                    E  R  T  Q  I  K  N  R  H  M  Y  L
```

30

```
      AACAGAAGAGATACTGAAAGAGAACCCTAACATGTGCGCATATAAGGCACCGTCGTTGGA
601   ---------+---------+---------+---------+---------+---------+  660
      TTGTCTTCTCTATGACTTTCTCTTGGGATTGTACACGCGTATATTCCGTGGCAGCAACCT

       T  E  E  I  L  K  E  N  P  N  M  C  A  Y  K  A  P  S  L  D  -

      TGCAAGAGAAGACATGATGATCAGGGAGGTACCAAGGGTTGGAAAAGAGGCTGCAACCAA
661   ---------+---------+---------+---------+---------+---------+  720
      ACGTTCTCTTCTGTACTACTAGTCCCTCCATGGTTCCCAACCTTTTCTCCGACGTTGGTT

       A  R  E  D  M  M  I  R  E  V  P  R  V  G  K  E  A  A  T  K  -

      GGCCATCAAGGAATGGGGCCAGCCAATGTCTAAGATCACACATTTGATCTTCTGCACCAC
721   ---------+---------+---------+---------+---------+---------+  780
      CCGGTAGTTCCTTACCCCGGTCGGTTACAGATTCTAGTGTGTAAACTAGAAGACGTGGTG

       A  I  K  E  W  G  Q  P  M  S  K  I  T  H  L  I  F  C  T  T  -

      CAGCGGCGTTGCGTTGCCTGGCGTTGATTATGAACTCATCGTACTCTTAGGGCTCGACCC
781   ---------+---------+---------+---------+---------+---------+  840
      GTCGCCGCAACGCAACGGACCGCAACTAATACTTGAGTAGCATGAGAATCCCGAGCTGGG

       S  G  V  A  L  P  G  V  D  Y  E  L  I  V  L  L  G  L  D  P  -

      AAGTGTCAAGAGGTACATGATGTACCACCAAGGTTGCTTTGCTGGTGGCACTGTCCTTCG
841   ---------+---------+---------+---------+---------+---------+  900
      TTCACAGTTCTCCATGTACTACATGGTGGTTCCAACGAAACGACCACCGTGACAGGAAGC

       S  V  K  R  Y  M  M  Y  H  Q  G  C  F  A  G  G  T  V  L  R  -

      TTTGGCTAAGGACTTGGCAGAAAACAACAAGGATGCTCGTGTGCTTATTGTTTGTTCTGA
901   ---------+---------+---------+---------+---------+---------+  960
      AAACCGATTCCTGAACCGTCTTTTGTTGTTCCTACGAGCACACGAATAACAAACAAGACT

       L  A  K  D  L  A  E  N  N  K  D  A  R  V  L  I  V  C  S  E  -
                         P
                         s
                         t
                         I
      GAATACTGCAGTCACTTTTCGTGGTCCTAATGAGACAGACATGGATAGTCTTGTAGGGCA
961   ---------+---------+---------+---------+---------+---------+  1020
      CTTATGACGTCAGTGAAAAGCACCAGGATTACTCTGTCTGTACCTATCAGAACATCCCGT

       N  T  A  V  T  F  R  G  P  N  E  T  D  M  D  S  L  V  G  Q  -

      AGCATTGTTTGCCGATGGAGCTGCTGCAATTATCATTGGTTCTGATCCTGTTCCAGAGGT
1021  ---------+---------+---------+---------+---------+---------+  1080
      TCGTAACAAACGGCTACCTCGACGACGTTAATAGTAACCAAGACTAGGACAAGGTCTCCA

       A  L  F  A  D  G  A  A  A  I  I  I  G  S  D  P  V  P  E  V  -

      TGAGAATCCTCTCTTTGAGATTGTTTCAACTGATCAACAACTTGTCCCTAACAGCCATGG
1081  ---------+---------+---------+---------+---------+---------+  1140
      ACTCTTAGGAGAGAAACTCTAACAAAGTTGACTAGTTGTTGAACAGGGATTGTCGGTACC

       E  N  P  L  F  E  I  V  S  T  D  Q  Q  L  V  P  N  S  H  G  -

      AGCCATCGGTGGTCTCCTTCGTGAAGTTGGACTTACATTTTATCTTAACAAGAGTGTTCC
1141  ---------+---------+---------+---------+---------+---------+  1200
      TCGGTAGCCACCAGAGGAAGCACTTCAACCTGAATGTAAAATAGAATTGTTCTCACAAGG

       A  I  G  G  L  L  R  E  V  G  L  T  F  Y  L  N  K  S  V  P  -
```

31

EP 0 309 862 B1

```
                                                        H
                                                        i
                                                        n
                                                        d
                                                        I
                                                        I
                                                        I
          GGATATTATTTCACAAAACATCAATGGTGCACTCAGTAAAGCTTTTGATCCACTGGGTAT
     1201 ---------+---------+---------+---------+---------+---------+ 1260
          CCTATAATAAAGTGTTTTGTAGTTACCACGTGAGTCATTTCGAAAACTAGGTGACCCATA

           D   I   I   S   Q   N   I   N   G   A   L   S   K   A   F   D   P   L   G   I   -

          ATCTGATTATAACTCAATATTTTGGATTGCACATCTTGGTGGACGCGCAATTTTGGACCA
     1261 ---------+---------+---------+---------+---------+---------+ 1320
          TAGACTAATATTGAGTTATAAAACCTAACGTGTAGAACCACCTGCGCGTTAAAACCTGGT

           S   D   Y   N   S   I   F   W   I   A   H   L   G   G   R   A   I   L   D   Q   -

          AGTTGAACAGAAGGTGAACTTGAAGCCAGAGAAGATGAAAGCCACTAGAGATGTACTTAG
     1321 ---------+---------+---------+---------+---------+---------+ 1380
          TCAACTTGTCTTCCACTTGAACTTCGGTCTCTTCTACTTTCGGTGATCTCTACATGAATC

           V   E   Q   K   V   N   L   K   P   E   K   M   K   A   T   R   D   V   L   S   -

          CAATTATGGTAACATGTCAAGTGCGTGTGTGTTCTTCATTATGGATTTGATGAGAAAGAA
     1381 ---------+---------+---------+---------+---------+---------+ 1440
          GTTAATACCATTGTACAGTTCACGCACACACAAGAAGTAATACCTAAACTACTCTTTCTT

           N   Y   G   N   M   S   S   A   C   V   F   F   I   M   D   L   M   R   K   K   -

          GTCACTTGAAACTGGACTTAAAACCACTGGAGAAGGACTTGATTGGGGTGTGTTGTTTGG
     1441 ---------+---------+---------+---------+---------+---------+ 1500
          CAGTGAACTTTGACCTGAATTTTGGTGACCTCTTCCTGAACTAACCCCACACAACAAACC

           S   L   E   T   G   L   K   T   T   G   E   G   L   D   W   G   V   L   F   G   -

          TTTTGGCCCTGGTCTCACTATTGAAACCGTTGTTCTCCGCAGCATGGCCATAtaatacgc
     1501 ---------+---------+---------+---------+---------+---------+ 1560
          AAAACCGGGACCAGAGTGATAACTTTGGCAACAAGAGGCGTCGTACCGGTATattatgcg

           F   G   P   G   L   T   I   E   T   V   V   L   R   S   M   A   I   *

          ttaattatatatctctgcatatatgcaattttgttattttttaataattttcttttactc
     1561 ---------+---------+---------+---------+---------+---------+ 1620
          aattaatatatagagacgtatatacgttaaaacaataaaaaattattaaaagaaaatgag

          taaaataagattctaaatggcttatattcttagatgagtgaaaacttagacagagatgtc
     1621 ---------+---------+---------+---------+---------+---------+ 1680
          attttattctaagatttaccgaatataagaatctactcacttttgaatctgtctctacag

          taaagttaattcgttatgcgaaga
     1681 ---------+---------+---- 1704
          atttcaattaagcaatacgcttct
```

8.  DNA consisting of or recombinant DNA containing the DNA sequence of the encoding DNA region according to Claim 7, including the DNA sequences derived therefrom with stilbene synthase activity.

9.  Stilbene synthase gene or gene unit containing the DNA according to Claim 7.

10. Recombinant prokaryotic or eukaryotic DNA, which contains one or more stilbene synthase genes or gene units and/or their structural genes or their regulatory gene parts and/or the DNA according to Claims 1 to 9 as "foreign" DNA or as "additional" DNA.

11. Recombinant DNA according to Claim 10, which is contained in plant cells (including protoplasts) or plants (including parts of plants and seeds).

32

**12.** Vectors, which contain one or more stilbene synthase genes or gene units or their structural genes or their regulatory gene parts and/or the DNA according to Claims 1 to 9 and/or the recombinant DNA according to Claim 10.

**13.** Vector plasmid pGS 828.1 (obtainable from E. coli DSM 4243).

**14.** Transformed microorganisms, which contain one or more stilbene synthase genes or gene units and/or their structural genes or their regulatory gene parts and/or the DNA according to Claims 1 to 9 and/or the recombinant DNA according to Claim 10.

**15.** Escherichia coli strain Nurdug 2010 (corresponding to E. coli DSM 4243) and its mutants and variants.

**16.** Use of the stilbene synthase gene or the gene unit and/or its structural gene or its regulatory gene part and/or the DNA and/or the recombinant DNA and/or the vectors and/or the transformed microorganisms according to Claims 1 to 15 for the transformation of plant cells (including protoplasts) and plants (including parts of plants and seeds).

**17.** Transformed plant cells (including protoplasts) and plants (including parts of plants and seeds), which contain one or more stilbene synthase genes or gene units and/or their structural genes or their regulatory gene parts and/or the DNA and/or the recombinant DNA according to Claims 1 to 10 as "foreign" or "additional" DNA.

**18.** Process for the preparation of transformed plant cells (including protoplasts) and plants (including parts of plants and seeds) according to Claim 17, characterised in that
(a) one or more stilbene synthase genes or gene units and/or their structural genes or their regulatory gene parts and/or the DNA and/or the recombinant DNA according to Claims 1 to 10 are introduced into the genome of plant cells (including protoplasts), and, if appropriate,
(b) complete transformed plants are regenerated from the transformed plant cells (including protoplasts), and, if appropriate,
(c) the desired parts of the plants (including seeds) are recovered from the transformed plants thus obtained.

**19.** Use of transformed plant cells (including protoplasts) according to Claim 17 for the production of propagation material and for the production of novel plants and propagation material thereof.

**20.** Propagation material, which can be obtained by the propagation of the transformed plant cells and plants according to Claim 17.

**21.** Transformed plant cells (including protoplasts) and plants (including parts of plants and seeds) according to Claim 17, the process for their preparation according to Claim 18, and their use according to Claim 19, and also the propagation material according to Claim 20, in which the plant cells and plants are tobacco, lucerne or potato plant cells and tobacco, lucerne or potato plants.

**Revendications**

**1.** Gène de la stilbène-synthétase, correspondant au gène de la stilbène-synthétase contenu dans le plasmide pGS 828.1 (qu'il est possible d'obtenir à partir de E. coli DSM 4243) ainsi que les séquences d'ADN à activité stilbène-synthétase en dérivant.

**2.** Gène de la stilbène-synthétase selon la revendication 1, la stilbène-synthétase désignant la resvératrol-synthétase et la pinosylvine-synthétase.

**3.** Gène de la stilbène-synthétase selon les revendications 1 et 2, qu'il est possible d'obtenir à partir d'arachides (Arachis hypogaea), de Pinus et de vigne.

**4.** Unité génique de la stilbène-synthétase, composée d'environ 6700 paires de bases et présentant 3 sites de coupure EcoRI, 3 sites de coupure HindIII et 1 site de coupure PstI et qu'il est possible d'obtenir par coupure partielle du plasmide pGS 828.1 (qu'il est possible d'obtenir à partir de E. coli DSM 4243) par EcoRI et HindIII ou à partir du plasmide pGS 828.1 (qu'il est possible d'obtenir à partir de E. coli DSM 4243) à l'aide de SstI et PvuII.

5. Partie à effet de régulation du gène de la stilbène-synthétase selon les revendications 1 à 4.

6. Gène de structure du gène de la stilbène-synthétase selon les revendications 1 à 4.

7. ADN présentant la séquence suivante pouvant se présenter avec ou sans intron, ainsi que les séquences d'ADN à activité stilbène-synthétase en dérivant.

```
                                    E
                                    c
                                    o
                                    R
                                    I
      cacagctaagaaaagATGGTGTCTGTGAGTGGAATTCGCAAGGTTCAAAGGGCAGAAGGT
    1 ---------+---------+---------+---------+---------+---------+ 60
      gtgtcgattcttttcTACCACAGACACTCACCTTAAGCGTTCCAAGTTTCCCGTCTTCCA

                 M   V   S   V   S   G   I   R   K   V   Q   R   A   E   G   -
      CCAGCAACGGTATTGGCGATCGGAACAGCAAATCCACCAAACTGTGTTGATCAGAGTACA
   61 ---------+---------+---------+---------+---------+---------+ 120
      GGTCGTTGCCATAACCGCTAGCCTTGTCGTTTAGGTGGTTTGACACAACTAGTCTCATGT

             P   A   T   V   L   A   I   G   T   A   N   P   P   N   C   V   D   Q   S   T   -
      TATGCAGATTATTATTTTAGAGTAACCAACGGCGAACACATGACTGATCTCAAGAAGAAA
  121 ---------+---------+---------+---------+---------+---------+ 180
      ATACGTCTAATAATAAAATCTCATTGGTTGCCGCTTGTGTACTGACTAGAGTTCTTCTTT

             Y   A   D   Y   Y   F   R   V   T   N   G   E   H   M   T   D   L   K   K   K   -
      TTTCAGCGCATCTgtatgtattttttattaagcgttctatatttgtttatatttaatattt
  181 ---------+---------+---------+---------+---------+---------+ 240
      AAAGTCGCGTAGAcatacataaaaataattcgcaagatataaacaaatataaattataaa

      F   Q   R   I   C

      atcaaaataaaattcgttctttatttttatattaattaatacaggaataatttaacata
  241 ---------+---------+---------+---------+---------+---------+ 300
      tagtttattttaagcaagaaaataaaaatataattaattatgtccttattaaaattgtat

      ttatatacaacatatcatattggctacttaatattaacaataataattacttaataataa
  301 ---------+---------+---------+---------+---------+---------+ 360
      aatatatgttgtatagtataaccgatgaattataattgttattattaatgaattattatt

      attatttcaatagttatataataaattatttcaattcttatacatttgcataaactatta
  361 ---------+---------+---------+---------+---------+---------+ 420
      taataaagttatcaatattttatttaataaagttaagaatatgtaaacctatttgataat

      aaataatgacacatgaacgttcaagttactataaaaataactcaaaataacattattat
  421 ---------+---------+---------+---------+---------+---------+ 480
      tttattactctgtacttgcaagttcaatgatattttttattgagttttttattgtaataata

      tgatatgtgtgtgtatgtgtatgtcgttttttctaaatgtcatcaagcgtattcatggatg
  481 ---------+---------+---------+---------+---------+---------+ 540
      actatacacacacatacacatacagcaaaaagatttacagtagttcccataactacctac

      tgaatttcatattattatttcagGTGAGAGAACACAGATCAAGAATAGACATATGTACTT
  541 ---------+---------+---------+---------+---------+---------+ 600
      acttaaagtataataataaagtcCACTCTCTTGTGTCTAGTTCTTATCTGTATACATGAA

                 E   R   T   Q   I   K   N   R   H   M   Y   L
```

```
     AACAGAAGAGATACTGAAAGAGAACCCTAACATGTGCGCATATAAGGCACCGTCGTTGGA
601  ---------+---------+---------+---------+---------+---------+  660
     TTGTCTTCTCTATGACTTTCTCTTGGGATTGTACACGCGTATATTCCGTGGCAGCAACCT

       T   E   I   L   K   E   N   P   N   M   C   A   Y   K   A   P   S   L   D   -


     TGCAAGAGAAGACATGATGATCAGGGAGGTACCAAGGGTTGGAAAAGAGGCTGCAACCAA
661  ---------+---------+---------+---------+---------+---------+  720
     ACGTTCTCTTCTGTACTACTAGTCCCTCCATGGTTCCCAACCTTTTCTCCGACGTTGGTT

       A   R   E   D   M   M   I   R   E   V   P   R   V   G   K   E   A   A   T   K   -


     GGCCATCAAGGAATGGGGCCAGCCAATGTCTAAGATCACACATTTGATCTTCTGCACCAC
721  ---------+---------+---------+---------+---------+---------+  780
     CCGGTAGTTCCTTACCCCGGTCGGTTACAGATTCTAGTGTGTAAACTAGAAGACGTGGTG

       A   I   K   E   W   G   Q   P   M   S   K   I   T   H   L   I   F   C   T   T   -


     CAGCGGCGTTGCGTTGCCTGGCGTTGATTATGAACTCATCGTACTCTTAGGGCTCGACCC
781  ---------+---------+---------+---------+---------+---------+  840
     GTCGCCGCAACGCAACGGACCGCAACTAATACTTGAGTAGCATGAGAATCCCGAGCTGGG

       S   G   V   A   L   P   G   V   D   Y   E   L   I   V   L   L   G   L   D   P   -


     AAGTGTCAAGAGGTACATGATGTACCACCAAGGTTGCTTTGCTGGTGGCACTGTCCTTCG
841  ---------+---------+---------+---------+---------+---------+  900
     TTCACAGTTCTCCATGTACTACATGGTGGTTCCAACGAAACGACCACCGTGACAGGAAGC

       S   V   K   R   Y   M   M   Y   H   Q   G   C   F   A   G   G   T   V   L   R   -


     TTTGGCTAAGGACTTGGCAGAAAACAACAAGGATGCTCGTGTGCTTATTGTTTGTTCTGA
901  ---------+---------+---------+---------+---------+---------+  960
     AAACCGATTCCTCAACCGTCTTTTGTTGTTCCTACGAGCACACGAATAACAAACAAGACT

       L   A   K   D   L   A   E   N   N   K   D   A   R   V   L   I   V   C   S   E   -

                             P
                             s
                             t
                             I
     GAATACTGCAGTCACTTTTCGTGGTCCTAATGAGACAGACATGGATAGTCTTGTAGGGCA
961  ---------+---------+---------+---------+---------+---------+  1020
     CTTATGACGTCAGTGAAAAGCACCAGGATTACTCTGTCTGTACCTATCAGAACATCCCGT

       N   T   A   V   T   F   R   G   P   N   E   T   D   M   D   S   L   V   G   Q   -


     AGCATTGTTTGCCGATGGAGCTGCTGCAATTATCATTGGTTCTGATCCTGTTCCAGAGGT
1021 ---------+---------+---------+---------+---------+---------+  1080
     TCGTAACAAACGGCTACCTCGACGACGTTAATAGTAACCAAGACTAGGACAAGGTCTCCA

       A   L   F   A   D   G   A   A   A   I   I   I   G   S   D   P   V   P   E   V   -


     TGAGAATCCTCTCTTTGAGATTGTTTCAACTGATCAACAACTTGTCCCTAACAGCCATGG
1081 ---------+---------+---------+---------+---------+---------+  1140
     ACTCTTAGGAGAGAAACTCTAACAAAGTTGACTAGTTGTTGAACAGGGATTGTCGGTACC

       E   N   P   L   F   E   I   V   S   T   D   Q   Q   L   V   P   N   S   H   G   -


     AGCCATCGGTGGTCTCCTTCGTGAAGTTGGACTTACATTTTATCTTAACAAGAGTGTTCC
1141 ---------+---------+---------+---------+---------+---------+  1200
     TCGGTAGCCACCAGAGGAAGCACTTCAACCTGAATGTAAAATAGAATTGTTCTCACAAGG

       A   I   G   G   L   L   R   E   V   G   L   T   F   Y   L   N   K   S   V   P   -
```

35

```
                                            H
                                            i
                                            n
                                            d
                                            I
                                            I
                                            I
      GGATATTATTTCACAAAACATCAATGGTGCACTCAGTAAAGCTTTTGATCCACTGGGTAT
1201  ---------+---------+---------+---------+---------+---------+ 1260
      CCTATAATAAAGTGTTTTGTAGTTACCACGTGAGTCATTTCGAAAACTAGGTGACCCATA

       D   I   I   S   Q   N   I   N   G   A   L   S   K   A   F   D   P   L   G   I   -

      ATCTGATTATAACTCAATATTTTGGATTGCACATCTTGGTGGACGCGCAATTTTGGACCA
1261  ---------+---------+---------+---------+---------+---------+ 1320
      TAGACTAATATTGAGTTATAAAACCTAACGTGTAGAACCACCTGCGCGTTAAAACCTGGT

       S   D   Y   N   S   I   F   W   I   A   H   L   G   G   R   A   I   L   D   Q   -

      AGTTGAACAGAAGGTGAACTTGAAGCCAGAGAAGATGAAAGCCACTAGAGATGTACTTAG
1321  ---------+---------+---------+---------+---------+---------+ 1380
      TCAACTTGTCTTCCACTTGAACTTCGGTCTCTTCTACTTTCGGTGATCTCTACATGAATC

       V   E   Q   K   V   N   L   K   P   E   K   M   K   A   T   R   D   V   L   S   -

      CAATTATGGTAACATGTCAAGTGCGTGTGTGTTCTTCATTATGGATTTGATGAGAAAGAA
1381  ---------+---------+---------+---------+---------+---------+ 1440
      GTTAATACCATTGTACAGTTCACGCACACACAAGAAGTAATACCTAAACTACTCTTTCTT

       N   Y   G   N   M   S   S   A   C   V   F   F   I   M   D   L   M   R   K   K   -

      GTCACTTGAAACTGGACTTAAAACCACTGGAGAAGGACTTGATTGGGGTGTGTTGTTTGG
1441  ---------+---------+---------+---------+---------+---------+ 1500
      CAGTGAACTTTGACCTGAATTTTGGTGACCTCTTCCTGAACTAACCCCACACAACAAACC

       S   L   E   T   G   L   K   T   T   G   E   G   L   D   W   G   V   L   F   G   -

      TTTTGGCCCTGGTCTCACTATTGAAACCGTTGTTCTCCGCAGCATGGCCATAtaatacgc
1501  ---------+---------+---------+---------+---------+---------+ 1560
      AAAACCGGGACCAGAGTGATAACTTTGGCAACAAGAGGCGTCGTACCGGTATattatgcg

       F   G   P   G   L   T   I   E   T   V   V   L   R   S   M   A   I   *

      ttaattatatatctctgcatatatgcaattttgttattttttaataattttcttttactc
1561  ---------+---------+---------+---------+---------+---------+ 1620
      aattaatatatagagacgtatatacgttaaaacaataaaaaattattaaaagaaaatgag

      taaaataagattctaaatggcttatattcttagatgagtgaaaacttagacagagatgtc
1621  ---------+---------+---------+---------+---------+---------+ 1680
      attttattctaagatttaccgaatataagaatctactcacttttgaatctgtctctacag

      taaagttaattcgttatgcgaaga
1681  ---------+---------+---- 1704
      atttcaattaagcaatacgcttct
```

8. ADN composé de la séquence d'ADN de la région codante de l'ADN selon la revendication 7 ou ADN recombiné contenant la séquence d'ADN de la région codante de l'ADN selon la revendication 7, dont les séquences d'ADN à activité stilbène-synthétase en dérivant.

9. Gène ou unité génique de la stilbène-synthétase contenant l'ADN selon la revendication 7.

10. ADN procaryote ou eucaryote recombiné contenant un ou plusieurs gènes ou unités géniques de la stilbène-synthétase et/ou leurs gènes de structure ou leurs parties géniques de régulation et/ou l'ADN selon les revendications 1 à 9 en tant qu'ADN "étranger" ou ADN "additionnel".

11. ADN recombinant selon la revendication 10, contenu dans des cellules végétales (dont des protoplas-

EP 0 309 862 B1

tes) ou des plantes (dont des parties de plantes et des graines).

12. Vecteurs contenant un ou plusieurs gènes ou unités géniques de la stilbène-synthétase ou leurs gènes de structure ou leurs parties géniques de régulation et/ou l'ADN selon les revendications 1 à 9 et/ou l'ADN recombiné selon la revendication 10.

13. Plasmide-vecteur pGS 828.1 (qu'il est possible d'obtenir à partir de E. coli DSM 4243).

14. Micro-organismes transformés contenant un ou plusieurs gènes ou unités géniques de la stilbène-synthétase et/ou leurs gènes de structure ou leurs parties géniques de régulation et/ou l'ADN selon les revendications 1 à 9 et/ou l'ADN recombiné selon la revendication 10.

15. Souche Nurdug 2010 de Escherichia coli (correspondant à E. coli DSM 4243) ainsi que ses mutants et variants.

16. Emploi du gène ou de l'unité génique de la stilbène-synthétase et/ou de leur gène de structure ou de leur partie génique de régulation et/ou de l'ADN et/ou de l'ADN recombiné et/ou des vecteurs et/ou des micro-organismes transformés selon les revendications 1 à 15 en vue de la transformation de cellules végétales (dont des protoplastes) et de plantes (dont des parties de plantes et des graines).

17. Cellules végétales (dont des protoplastes) et plantes (dont des parties de plantes et des graines) transformées contenant un ou plusieurs gènes ou unités géniques de la stilbène-synthétase et/ou leurs gènes de structure ou leurs parties géniques de régulation et/ou l'ADN et/ou l'ADN recombiné selon les revendications 1 à 10 en tant qu'ADN "étranger" ou "additionnel".

18. Procédé de préparation de cellules végétales (dont des protoplastes) et de plantes (dont des parties de plantes et des graines) transformées selon la revendication 17, caractérisé en ce que
(a) on intègre un ou plusieurs gènes ou unités géniques de la stilbène-synthétase et/ou leurs gènes de structure ou leurs parties géniques de régulation et/ou l'ADN et/ou l'ADN recombiné selon les revendications 1 à 10 dans le génome de cellules végétales (dont des protoplastes) et le cas échéant
(b) on régénère des plantes transformées entières à partir des cellules végétales transformées (dont des protoplastes) et le cas échéant
(c) on obtient les parties de plantes souhaitées (dont des graines) à partir des plantes transformées ainsi obtenues.

19. Emploi de cellules végétales (dont des protoplastes) transformées selon la revendication 17 pour la production d'un matériel de multiplication ainsi que pour la production de plantes nouvelles et de leur matériel de multiplication.

20. Matériel de multiplication, qu'il est possible d'obtenir par la multiplication des cellules végétales et plantes transformées selon la revendication 17.

21. Cellules végétales (dont des protoplastes) et plantes (dont des parties de plantes et des graines) transformées selon la revendication 17, leur procédé de préparation selon la revendication 18 et leur emploi selon la revendication 19 ainsi que le matériel de multiplication selon la revendication 20, les cellules végétales et les plantes étant des cellules végétales de tabac, de luzerne ou de pomme de terre et des plantes de tabac, de luzerne ou de pomme de terre.

37

FIG.1

FIG.2